# EUROPEAN PATENT SPECIFICATION

(11) **EP 2 595 970 B1**
(45) Date of publication and mention of the grant of the patent: **07.01.2015**
(21) Application number: 11746655.7
(22) Date of filing: 22.07.2011
(51) Int. Cl.: C07D 271/06, C07D 237/20, A61K 31/425, A61P 3/00

(54) **DERIVATIVES OF OXADIAZOLE AND PYRIDAZINE, THEIR PREPARATION AND THEIR APPLICATION IN THERAPEUTICS**
OXADIAZOL- UND PYRIDAZINDERIVATE, IHRE HERSTELLUNG UND THERAPEUTISCHE ANWENDUNG
DÉRIVÉS D'OXADIAZOLE ET DE PYRIDAZINE, LEUR PRÉPARATION ET LEUR APPLICATION EN THÉRAPIE

(30) Priority: 23.07.2010 FR 1056060
(43) Date of publication of application: 29.05.2013
(73) Proprietor: SANOFI, 75013 Paris (FR)
(72) Inventor: FETT, Eykmar, F-75013 Paris (FR); MOUGENOT, Patrick, F-75013 Paris (FR); NAMANE, Claudie, F-75013 Paris (FR); NICOLAI, Eric, F-75013 Paris (FR); PHILIPPO, Christophe, F-75013 Paris (FR)
(74) Representative: Romanowski, Caroline
(86) International application number: PCT/IB2011/053278
(87) International publication number: WO 2012/011081

(56) References cited:
- WO-A1-2010/086551
- FR-A1- 2 941 457
- CHEN ET AL: "Inhibition of Triglyceride Synthesis as a Treatment Strategy for Obesity", ARTHERIOSCLER. THROMB. VASC. BIOL,, vol. 25, 29 November 2004 (2004-11-29), pages 482-486, XP008115583, DOI: 10.1161/01.ATV.0000151874.81059.AD

## Description

The present invention relates to oxadiazole and pyridazine derivatives, to the preparation thereof and to the therapeutic use thereof. The present compounds of the invention inhibit the synthesis of triglycerides and are of use for the treatment of pathologies in which such inhibition is beneficial, such as in the case of obesity, dyslipidemia, non-alcoholic hepatic steatosis, non-insulin-dependent type 2 diabetes, metabolic syndrome and acne.

Triacylglycerides represent the main form of energy storage in eukaryotes, and may also be the cause of disorders or imbalances in the metabolism of triacylglycerides, involved in the pathogenesis and the increase of risk of several pathologies such as obesity, insulin resistance, type 2 diabetes (Reasner C.A., J. Cardiovasc. Pharmacol. 52:136-44, 2008) and complications arising from this pathology (Krane and Wanner, Minerva Urol. Nefrol. 59(3):299-316, 2007; King G.L., J. Periodontol. 79:1527-34, 2008), dyslipidemia, which is characterized by high levels of plasmatic triglycerides, low levels of high-density lipoproteins (HDL) and the appearance of small dense low-density lipoproteins (sdLDL) and excessive postprandial lipidemia (Ginsberg et al., Obesity (Silver Spring). 14: 41S-49S, 2006, Adiels et al., Curr. Opin. Lipidol. 17: 238-246, 2006, Adiels et al., ATVB 28:1225-1236,2008), impaired fasting glucose conditions, metabolic acidosis, ketosis, metabolic syndrome (Eschwège E. Diabetes Metab. 29:6S19-27, 2003), hepatic steatosis (Parekh and Anania, Gastroenterology 132:2191-2207, 2007), coronary diseases (Lewis et al., Endocrine Review 23:701, 2002; Ridker and Silvertown, J. Periodontol., 79:1544-51, 2008; McBride P. Curr. Atheroscler. Rep. 10:386-90, 2008), skin diseases (Chen et al., J. Clin. Invest., 109:175-81, 2002; Yosipovitch et al., J. Am. Acad. Dermatol., 56:901-16, 2007), Alzheimer's disease, various immunomodulatory diseases (Pahan K., Cell Mol. Life Sci., 63:1165-78, 2006), HIV infection (Kotler D.P., J. Acquir. Immune Defic. Syndr., 49:S79-85, 2008), irritable bowel syndrome (Schäffler et al., Nat. Clin. Pract. Gastroenterol. Hepatol., 2:103-11, 2005). Excessive storage of triacylglycerides in lean tissues, such as the liver, the muscles and other peripheral tissues, leads to dysfunction in these tissues; whereas reducing the accumulation of these fats in these peripheral tissues appears to be beneficial in treating lipotoxicity (Unger, Endocrinology, 144: 5 159-5 165, 2003). The accumulation and an excess of triacylglycerides in adipose tissue (WAT) leads to obesity, a condition that is associated with a reduction in lifespan, type II diabetes, coronary diseases, hypertension, strokes, and the development of certain cancers (Grundy, Endocrine 13 (2): 155-165, 2000).

DGAT-1 catalyzes the final step of the synthesis of triglycerides (TGs), converting diacylglycerol and acyl-CoA into triglycerides. There are two isoforms of diacylglycerol acyltransferase: DGAT-1 (U.S. Pat. No. 6,100,077; Farese et al., Proc. Nat. Acad. Sci. 95:13018-13023, 1998) and DGAT-2 (Farese et al., J. Biol. Chem. 276: 38870-38876, 2001). DGAT-1 and DGAT-2 share 12% of the amino acid sequence.

A mouse deficient in DGAT-1 is healthy and fertile, and is capable of carrying out the biosynthesis of triglycerides (Farese et al. Nature Genetics 25: 87-90, 2000). A mouse deficient in DGAT-1 is resistant to diet-induced obesity, has an increased sensitivity to insulin (Farese et al. Nature Genetics 25: 87-90, 2000) and to leptin (Farese et al. J. Clin. Invest. 109:1049-1055, 2002). A mouse deficient in DGAT-1 shows a lower absorption rate of triglycerides and an improvement in the metabolism of triglycerides. After loading with glucose, a mouse deficient in DGAT-1 shows a lower glucose and insulin level than mice of wild phenotype, suggesting an improvement in the metabolism of glucose (Farese et al., J. Biol. Chem. 277: 25474-25479, 2002).

The synthesis of TGs, the size and the proliferation of the sebaceous glands decrease with inhibition of DGAT-1 in hamsters (J Invest Dermatol 127, 2740-48, 2007) clearly showing a therapy against skin diseases such as acne that involves this mechanism.

The inhibition of DGAT-1 by oligonucleotides reduces hepatic TGs suggesting a therapy against hepatic steatosis that involves this mechanism (Hepatology 50, 434-442,2009).

The activity of DGAT-1 in the host cell is required for the replication of the hepatitis C virus. This mechanism suggests a therapy against hepatitis C via inhibition of DGAT-1 (Nature Medecine (16)11, 1295-1298, 2011).

One subject of the present invention is compounds corresponding to the formula (I): 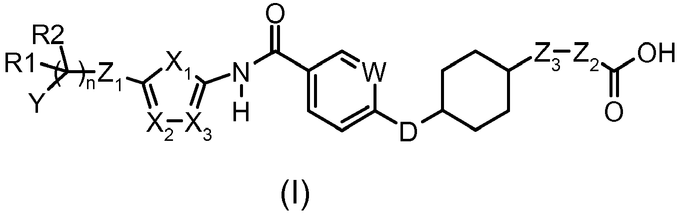 in which:
- **n** is equal to 0 or 1;
- **D** represents an oxygen atom or a bond;
- **W** represents a nitrogen atom or a -CH- group;
- **X1** represents a nitrogen atom or a -CH=CH- group;
- **X2** represents an oxygen atom or a nitrogen atom;
- **X3** represents an oxygen atom or a nitrogen atom; one of X1, X2, X3 being other than a nitrogen atom, X2 and X3 not being an oxygen atom at the same time;
- **R1, R2** are absent or represent,
   o independently of one another, a hydrogen atom or a (C1-C4)alkyl group,
   o R1 and R2 may form, with the carbon atom to which they are attached, a -(C3-C10)cycloalkyl- group;
- **Y** represents a -(C3-C10)cycloalkyl-, aryl or aryloxy group, said groups being optionally substituted with one or more substituents chosen from a halogen atom or a (C1-C6)alkoxy group;
- **Z1** is absent or represents an -NH- function;
- **Z2** is absent or represents a methylene group or a 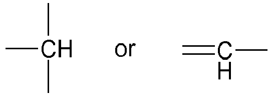 group;
- **Z3** is absent or represents an oxygen atom or a methylene group or a 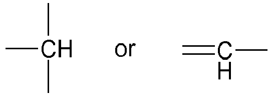 group;
   given that **Z2** only represents a 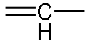 group when **Z3** is present and when it represents a 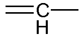 group, and vice versa, **Z2 and Z3** thus forming a double bond;
   given that **Z2** and **Z3,** when they are present, may be included in a cycloalkyl group;
   given that when **Z3** represents an oxygen atom, **Z2** represents a methylene group or a 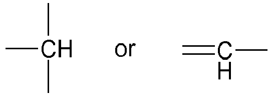 group;
in the form of an acid, a base or an addition salt with an acid or with a base.

Another subject of the present invention is compounds corresponding to the formula (I): 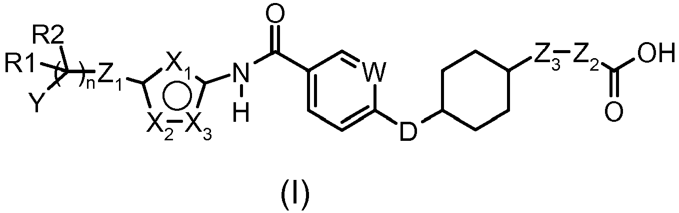 in which:
- **n** is equal to 0 or 1;
- **D** represents an oxygen atom or a bond;
- **W** represents a nitrogen atom or a -CH- group;
- **X1** represents a nitrogen atom or a -CH=CH- group;
- **X2** represents an oxygen atom or a nitrogen atom;
- **X3** represents an oxygen atom or a nitrogen atom; one of X1, X2, X3 being other than a nitrogen atom, X2 and X3 not being an oxygen atom at the same time;
- **R1, R2** are absent or represent,
   o independently of one another, a hydrogen atom or a (C1-C4)alkyl group,
   o R1 and R2 may form, with the carbon atom to which they are attached, a -(C3-C10)cycloalkyl- group;
- **Y** represents a -(C3-C10)cycloalkyl-, aryl or aryloxy group, said groups being optionally substituted with one or more substituents chosen from a halogen atom or a (C1-C6)alkoxy group;
- **Z1** is absent or represents an -NH- function;
- **Z2** is absent or represents a methylene group or a 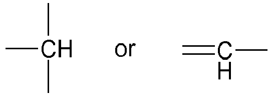 group;
- **Z3** is absent or represents an oxygen atom or a methylene group or a 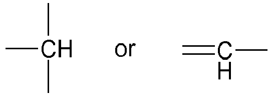 group;
   given that **Z2** only represents a 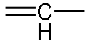 group when **Z3** is present and when it represents a 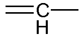 group, and vice versa, **Z2 and Z3** thus forming a double bond;
   given that **Z2** and **Z3,** when they are present, may be included in a cycloalkyl group;
   given that when **Z3** represents an oxygen atom, **Z2** represents a methylene group or a 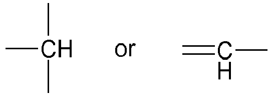 group;
in the form of an acid, a base or an addition salt with an acid or with a base.

Another subject of the present invention is compounds corresponding to the formula (I): 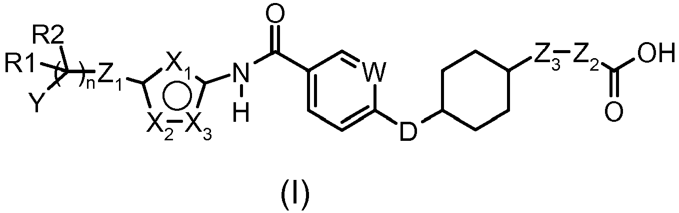 in which:
- **n** is equal to 0 or 1;
- **D** represents an oxygen atom or a bond;
- **W** represents a nitrogen atom or a -CH- group;
- **X1** represents a nitrogen atom or a -CH=CH- group;
- **X2** represents an oxygen atom or a nitrogen atom;
- **X3** represents an oxygen atom or a nitrogen atom; one of X1, X2, X3 being other than a nitrogen atom, X2 and X3 not being an oxygen atom at the same time;
- **R1, R2** are absent or represent,
   o independently of one another, a hydrogen atom or a (C1-C4)alkyl group,
   o R1 and R2 may form, with the carbon atom to which they are attached, a -(C3-C10)cycloalkyl- group;
- **Y** represents a -(C3-C10)cycloalkyl-, aryl or aryloxy group, said groups being optionally substituted with one or more substituents chosen from a halogen atom or a (C1-C6)alkoxy group;
- **Z1** is absent or represents an -NH- function;
- **Z2** is absent or represents a methylene group or a 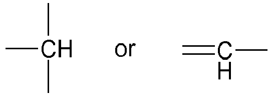 group;
- **Z3** is absent or represents an oxygen atom or a methylene group or a 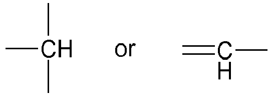 group;
   given that **Z2** only represents a 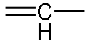 group when **Z3** is present and when it represents a 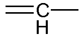 group, and vice versa, **Z2 and Z3** thus forming a double bond;
   given that **Z2** and **Z3,** when they are present, may be included in a cycloalkyl group;
   given that when **Z3** represents an oxygen atom, **Z2** represents a methylene group or a 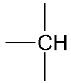 group;
   in the form of an acid, a base or an addition salt with an acid or with a base.

Of course, the ring comprising X1, X2, X3 is a heteroaryl group, the position of the double bonds possibly varying depending on the values of X1, X2, X3 in order to lead to an electron distribution corresponding to an aromatic ring.

The compounds of formula (I) may comprise one or more asymmetric carbon atoms. They may thus exist in the form of enantiomers or diastereoisomers. These enantiomers and diastereoisomers, and also mixtures thereof, including racemic mixtures, form part of the invention.

In the compounds of formula (I), the substituents borne by the cyclohexyl group may be in the cis or trans position. The compounds of formula (I) may thus exist in the form of positional isomers as defined previously. These positional isomers, and also a mixture thereof, form part of the invention.

The compounds of formula (I) may exist in the form of bases or salified with acids or bases, especially pharmaceutically acceptable acids or bases. Such addition salts form part of the invention.

These salts are advantageously prepared with pharmaceutically acceptable bases, but the salts of other bases that are useful, for example, for purifying or isolating the compounds of formula (I) also form part of the invention.

In the context of the present invention, and unless otherwise mentioned in the text, the following definitions apply:
- a halogen atom: a fluorine, a chlorine, a bromine or an iodine;
- an alkyl group: a saturated, linear or branched aliphatic group, which may contain 1 to 6 carbon atoms (C1-C6). Examples that may be mentioned include methyl, ethyl, propyl, isopropyl, butyl, isobutyl, *tert*-butyl and pentyl groups, etc.;
- an alkylene group: a saturated, linear or branched divalent alkyl group as above, which may contain 1 to 5 carbon atoms. Examples that may be mentioned include methylene, ethylene and propylene radicals;
- a cycloalkyl group: a cyclic alkyl group which may contain 3 to 10 carbon atoms. Examples that may be mentioned include cyclopropyl, methylcyclopropyl, cyclobutyl, cyclopentyl, cyclohexyl and adamantyl groups, etc.;
- an alkoxy group: a radical of formula O-alkyl, in which the alkyl group is as defined previously;
- an aryl group: a cyclic aromatic group containing 6 carbon atoms. Examples of aryl groups that may be mentioned include a phenyl group;
- an aryloxy group: a radical of formula O-aryl, in which the aryl group is as defined previously;
- a heteroaryl group: a cyclic aromatic group containing between 2 and 6 carbon atoms, in particular between 5 and 6 carbon atoms, and comprising at least one heteroatom, such as nitrogen, oxygen or sulfur. Examples of heteroaryl groups that may be mentioned include a pyridyl, oxadiazolyl and pyridazinyl group.

Among the compounds of formula (I) that are subjects of the invention, mention may be made of a group in which:
- **n** is equal to 0 or 1;
   and/or
- **D** represents an oxygen atom or a bond;
   and/or
- **W** represents a nitrogen atom or a -CH- group;
   and/or
- **X1** represents a nitrogen atom or a -CH=CH- group;
   and/or
- **X2** represents an oxygen atom or a nitrogen atom;
   and/or
- **X3** represents an oxygen atom or a nitrogen atom;
   one of X1, X2 and X3 being other than a nitrogen atom and X2 and X3 not being an oxygen atom at the same time;
   and/or
- **R1, R2** are absent or represent:
   o independently of one another, a hydrogen atom or a (C1-C4)alkyl group;
   o R1 and R2 may form, with the carbon atom to which they are attached, a -(C3-C10)cycloalkyl- group;
      and/or
- **Y** represents a -(C3-C10)cycloalkyl-, aryl or aryloxy group, said groups being optionally substituted with one or more substituents chosen from a halogen atom or a (C1-C6)alkoxy group;
   and/or
- **Z1** is absent or represents an -NH- function;
   and/or
- **Z2** is absent or represents a methylene group or a 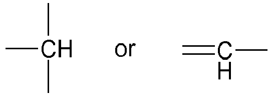 group;
   and/or
- Z3 is absent or represents an oxygen atom or a methylene group or a 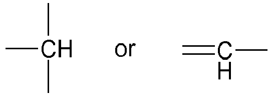 group;
   given that **Z2** only represents a 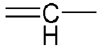 group when **Z3** is present and when it represents a 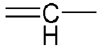 group, and vice versa, **Z2** and **Z3** thus forming a double bond;
   given that **Z2** and **Z3,** when they are present, may be included in a cycloalkyl group;
   given that when Z3 represents an oxygen atom, Z2 represents a methylene group
   or a 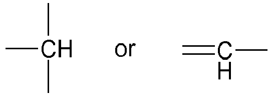 group;
   in the form of an acid or a base or an addition salt with an acid or with a base.

Among the compounds of formula (I) that are subjects of the invention, mention may be made of a group in which:
- **n** is equal to 0 or 1;
   and/or
- **D** represents an oxygen atom or a bond;
   and/or
- **W** represents a nitrogen atom or a -CH- group;
   and/or
- **X1** represents a nitrogen atom or a -CH=CH- group;
   and/or
- **X2** represents an oxygen atom or a nitrogen atom;
   and/or
- **X3** represents an oxygen atom or a nitrogen atom;
   one of X1, X2 and X3 being other than a nitrogen atom and X2 and X3 not being an oxygen atom at the same time;
   and/or
- **R1, R2** are absent or represent:
   o independently of one another, a hydrogen atom or a (C1-C4)alkyl group;
   o R1 and R2 may form, with the carbon atom to which they are attached, a -(C3-C10)cycloalkyl- group;
      and/or
- **Y** represents a -(C3-C10)cycloalkyl-, aryl or aryloxy group, said groups being optionally substituted with one or more substituents chosen from a halogen atom or a (C1-C6)alkoxy group;
   and/or
- **Z1** is absent or represents an -NH- function;
   and/or
- **Z2** is absent or represents a methylene group or a 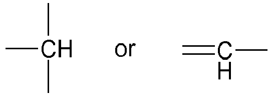 group;
   and/or
- Z3 is absent or represents an oxygen atom or a methylene group or a 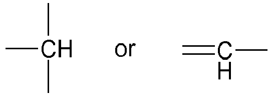 group;
   given that **Z2** only represents a 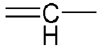 group when **Z3** is present and when it represents a 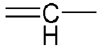 group, and vice versa, **Z2** and **Z3** thus forming a double bond;
   given that **Z2** and **Z3,** when they are present, may be included in a cycloalkyl group;
   given that when Z3 represents an oxygen atom, Z2 represents a methylene group or a 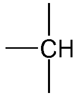 group;
   in the form of an acid or a base or an addition salt with an acid or with a base.

Among the latter compounds, mention may be made of those in which:
- **n** is equal to 0 or 1;
- **D** represents an oxygen atom or a bond;
- **W** represents a -CH- group;
- **X1** represents a nitrogen atom or a -CH=CH- group;
- **X2** represents an oxygen atom or a nitrogen atom;
- **X3** represents an oxygen atom or a nitrogen atom; one of X1, X2 and X3 being other than a nitrogen atom and X2 and X3 not being an oxygen atom at the same time;
- **R1, R2** are absent or represent:
   o independently of one another, a hydrogen atom or a (C1-C4)alkyl group;
   o R1 and R2 may form, with the carbon atom to which they are attached, a -(C3-C10)cycloalkyl- group;
- **Y** represents a -(C3-C10)cycloalkyl-, aryl or aryloxy group, said groups being optionally substituted with one or more substituents chosen from a halogen atom;
- **Z1** is absent or represents an -NH- function;
- **Z2** is absent;
- **Z3** is absent or represents a methylene group;
in the form of an acid or a base or an addition salt with an acid or with a base.

Among the latter compounds, mention may be made of those in which:
- n is equal to 1;
- **D** represents a bond;
- **W** represents a -CH- group;
- **X1** represents a nitrogen atom or a -CH=CH- group;
- **X2** represents an oxygen atom or a nitrogen atom;
- **X3** represents an oxygen atom or a nitrogen atom; one of X1, X2 and X3 being other than a nitrogen atom;
- **R1, R2** represent a hydrogen atom or a (C1-C4)alkyl group;
- **Y** represents an aryl group;
- **Z1** is absent;
- **Z2** is absent;
- **Z3** represents a methylene group;
in the form of an acid or a base or an addition salt with an acid or with a base.

Among the latter compounds, mention may be made of those in which:
- **n** is equal to 0 or 1;
- **D** represents an oxygen atom;
- **W** represents a -CH- group;
- **X1** represents a nitrogen atom or a -CH=CH- group;
- **X2** represents a nitrogen atom;
- **X3** represents an oxygen atom or a nitrogen atom; one of X1, X2 and X3 being other than a nitrogen atom;
- **R1, R2** represent:
   o independently of one another, a hydrogen atom or a (C1-C4)alkyl group;
   o R1 and R2 may form, with the carbon atom to which they are attached, a -(C3-C10)cycloalkyl- group;
- **Y** represents a -(C3-C10)cycloalkyl-, aryl or aryloxy group, said groups being optionally substituted with one or more halogen atoms;
- **Z1** is absent or represents an -NH- function;
- **Z2** is absent;
- **Z3** is absent;
in the form of an acid or a base or an addition salt with an acid or with a base.

Among the latter compounds, mention may be made of those in which:
- **n** is equal to 1;
- **D** represents a bond;
- **W** represents a -CH- group;
- **X1** represents a nitrogen atom;
- **X2** represents an oxygen atom;
- **X3** represents a nitrogen atom;
- **R1, R2** represent a hydrogen atom;
- **Y** represents an aryl group;
- **Z1** is absent;
- **Z2** is absent;
- **Z3** represents a methylene group;
in the form of an acid or a base or an addition salt with an acid or with a base.

Among the latter compounds, mention may be made of those in which:
- **n** is equal to 0 or 1;
- **D** represents an oxygen atom or a bond;
- **W** represents a -CH- group;
- **X1** represents a -CH=CH- group;
- **X2** represents a nitrogen atom;
- **X3** represents a nitrogen atom;
- **R1, R2** are absent or represent a hydrogen atom;
- **Y** represents a -(C3-C10)cycloalkyl- or aryl group;
- **Z1** is absent or represents an -NH- function;
- **Z2** is absent;
- **Z3** is absent or represents a methylene group;
in the form of an acid or a base or an addition salt with an acid or with a base.

Among the latter compounds, mention may be made of those in which:
- **n** is equal to 1;
- **D** represents an oxygen atom or a bond;
- **W** represents a -CH- group;
- **X1** represents a nitrogen atom;
- **X2** represents a nitrogen atom;
- **X3** represents an oxygen atom;
- **R1, R2** represent:
   o independently of one another, a hydrogen atom or a (C1-C4)alkyl group;
   o R1 and R2 may form, with the carbon atom to which they are attached, a -(C3-C10)cycloalkyl- group;
- **Y** represents an aryl or aryloxy group, said groups being optionally substituted with one or more substituents chosen from a halogen atom;
- **Z1** is absent;
- **Z2** is absent;
- **Z3** is absent or represents a methylene group;
in the form of an acid or a base or an addition salt with an acid or with a base.

Among the compounds of formula (I) that are subjects of the invention, mention may be made of a group in which n is equal to 0.

Among the compounds of formula (I) that are subjects of the invention, mention may be made of a group in which **n** is equal to 1.

Among the compounds of formula (I) that are subjects of the invention, mention may be made of a group in which **D** represents an oxygen atom.

Among the compounds of formula (I) that are subjects of the invention, mention may be made of a group in which **D** represents a bond.

Among the compounds of formula (I) that are subjects of the invention, mention may be made of a group in which **W** represents a -CH- group.

Among the compounds of formula (I) that are subjects of the invention, mention may be made of a group in which **W** represents a nitrogen atom.

Among the compounds of formula (I) that are subjects of the invention, mention may be made of a group in which **X1** represents a nitrogen atom.

Among the compounds of formula (I) that are subjects of the invention, mention may be made of a group in which **X1** represents a -CH=CH- group.

Among the compounds of formula (I) that are subjects of the invention, mention may be made of a group in which **X2** represents an oxygen atom.

Among the compounds of formula (I) that are subjects of the invention, mention may be made of a group in which **X2** represents a nitrogen atom.

Among the compounds of formula (I) that are subjects of the invention, mention may be made of a group in which **X3** represents an oxygen atom.

Among the compounds of formula (I) that are subjects of the invention, mention may be made of a group in which **X3** represents a nitrogen atom.

Among the compounds of formula (I) that are subjects of the invention, mention may be made of a group in which **X1** represents a nitrogen atom, **X2** and **X3** represent a nitrogen atom or an oxygen atom.

Among the compounds of formula (I) that are subjects of the invention, mention may be made of a group in which **X1** represents -CH=CH-, **X2** and **X3** represent a nitrogen atom.

Among the compounds of formula (I) that are subjects of the invention, mention may be made of a group in which **R1, R2** represent, independently of one another, a hydrogen atom or a (C1-C4)alkyl group.

Among the compounds of formula (I) that are subjects of the invention, mention may be made of a group in which **R1, R2** form, with the carbon atom to which they are attached, a -(C3-C10)cycloalkyl group, more particularly a cyclopropyl group.

Among the compounds of formula (I) that are subjects of the invention, mention may be made of a group in which **Y** represents a -(C3-C10)cycloalkyl group.

Among the compounds of formula (I) that are subjects of the invention, mention may be made of a group in which **Y** represents an aryl group, more particularly a phenyl group, said group being optionally substituted with one or more substituents chosen from a halogen atom, more particularly a fluorine atom or a (C1-C6)alkoxy group, more particularly a methoxy group.

Among the compounds of formula (I) that are subjects of the invention, mention may be made of a group in which **Y** represents an aryloxy group, more particularly a phenyloxy group.

Among the compounds of formula (I) that are subjects of the invention, mention may be made of a group in which **Z1** is absent.

Among the compounds of formula (I) that are subjects of the invention, mention may be made of a group in which **Z1** represents an -NH- function.

Among the compounds of formula (I) that are subjects of the invention, mention may be made of a group in which **Z2** is absent.

Among the compounds of formula (I) that are subjects of the invention, mention may be made of a group in which **Z2** represents a methylene group.

Among the compounds of formula (I) that are subjects of the invention, mention may be made of a group in which **Z2 and Z3** represent a 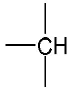 group, thus forming a cycloalkyl group.

Among the compounds of formula (I) that are subjects of the invention, mention may be made of a group in which **Z2 and Z3** represent a 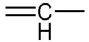 group, thus forming a double bond.

Among the compounds of formula (I) that are subjects of the invention, mention may be made of a group in which **Z3** is absent.

Among the compounds of formula (I) that are subjects of the invention, mention may be made of a group in which **Z3** represents an oxygen atom and **Z2** represents a methylene group or a 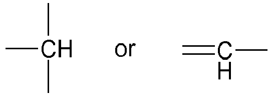 group.

Among the compounds of formula (I) that are subjects of the invention, mention may be made of a group in which **Z3** represents an oxygen atom and **Z2** represents a methylene group or a 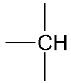 group.

Among the compounds of formula (I) that are subjects of the invention, mention may be made of a group in which **Z3** represents a methylene group.

Among the compounds of formula (I) that are subjects of the invention, mention may especially be made of the following compounds:
- trans{4-[4-(5-benzyl[1.2.4]oxadiazol-3-ylcarbamoyl)phenyl]cyclohexyl}acetic acid
- trans{4-[4-(3-benzyl[1.2.4]oxadiazol-5-ylcarbamoyl)phenyl]cyclohexyl}acetic acid
- cis-4-[4-(3-benzyl[1.2.4]oxadiazol-5-ylcarbamoyl)phenoxy]cyclohexane-carboxylic acid
- cis-4-{4-[3-(3,5-difluorobenzyl)[1.2.4]oxadiazol-5-ylcarbamoyl]phenoxy}cyclohexanecarboxylic acid
- cis-4-{4-[3-(1-phenylcyclopropyl)[1.2.4]oxadiazol-5-ylcarbamoyl]phenoxy}-cyclohexanecarboxylic acid
- cis-4-{4-[3-(1-methyl-1-phenylethyl[1.2.4]oxadiazol-5-ylcarbamoyl]phenoxy}-cyclohexanecarboxylic acid
- cis-4-[4-(3-phenoxymethyl[1.2.4]oxadiazol-5-ylcarbamoyl)phenoxy]cyclohexanecarboxylic acid
- {4-[4-(6-benzylpyridazin-3-ylcarbamoyl)phenyl]cyclohexyl}acetic acid
- cis-4-[4-(6-cyclopentylaminopyridazin-3-ylcarbamoyl)phenoxy]cyclohexane-carboxylic acid
- cis-4-[5-(3-benzyl[1.2.4]oxadiazol-5-ylcarbamoyl)pyridin-2-yloxy]cyclohexanecarboxylic acid
- trans-2-{4-[4-(3-benzyl[1.2.4]oxadiazol-5-ylcarbamoyl)phenyl]cyclohexyl}-cyclopropanecarboxylic acid
- trans-(E)-3-{4-[4-(3-benzyl[1.2.4]oxadiazol-5-ylcarbamoyl)phenyl]cyclohexyl}-acrylic acid
- trans-3-{4-[4-(3-benzyl[1.2.4]oxadiazol-5-ylcarbamoyl)phenyl]cyclohexyl}-propionic acid
- cis-4-[4-(6-phenylaminopyridazin-3-ylcarbamoyl)phenoxy]cyclohexanecarboxylic acid
- cis-4-{4-[6-(4-methoxyphenyl)pyridazin-3-ylcarbamoyl]phenoxy}cyclohexanecarboxylic acid

It should be noted that the above compounds were named in IUPAC nomenclature by means of the ACDLABS 10.0 ACD/name software (Advanced Chemistry Development).

In accordance with the invention, the compounds of general formula (I) may be prepared according to the following processes.

In the text hereinbelow, a "protecting group" (PG) is understood to mean a group that makes it possible, on the one hand, to protect a reactive function such as a hydroxyl or an amine during a synthesis and, on the other hand, to regenerate the intact reactive function at the end of the synthesis. Examples of protecting groups and of protection and deprotection methods are given in "Protective Groups in Organic Synthesis", Green et al., 4th Edition (John Wiley & Sons, Inc., New York).

In **schemes 1 to 11,** the starting compounds and the reactants, when their method of preparation is not described, are commercially available or are described in the literature, or else may be prepared according to methods that are described therein or that are known to those skilled in the art.

In accordance with the invention, the compounds of general formula (I) may be prepared according to the following processes:
The R group used below represents a -Z1-C(R1R2)ₙ-Y group with R1, R2, Y, n and Z1 as defined previously.

### Synthesis of the intermediates

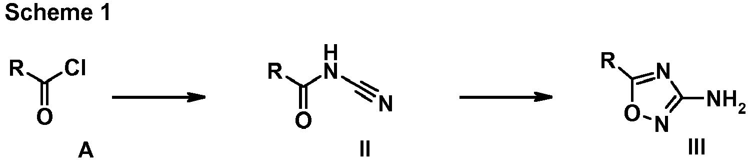

In scheme 1, the acid chloride of formula (A) for which R represents a benzyl group is reacted with cyanamide in a polar solvent such as THF in order to give the acylcyanamide of formula (II). This is reacted with hydroxylamine hydrochloride in the presence of a base such as pyridine in a protic solvent such as ethanol in order to result in the aminooxadiazole of formula (III).

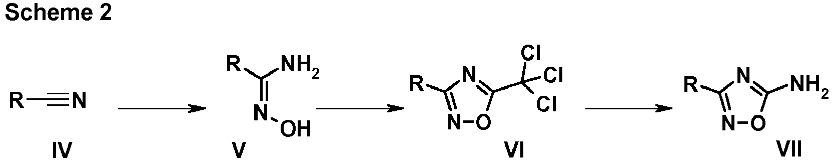

In **scheme 2,** the acetonitrile of formula (IV) for which R represents a benzyl, 3,5-difluorobenzyl, phenyldimethylmethyl or phenylcyclopropyl group is reacted with hydroxylamine hydrochloride in the presence of a base such as triethylamine in a polar solvent such as dichloromethane in order to give the hydroxyamidine of formula (V). The latter is reacted with trichloroacetyl chloride in the presence of a base such as pyridine in an apolar solvent such as toluene in order to give the trichloromethyloxadiazole of formula (VI). This, reacted with aqueous ammonia in a protic solvent such as methanol gives the aminooxadiazole of formula (VII).

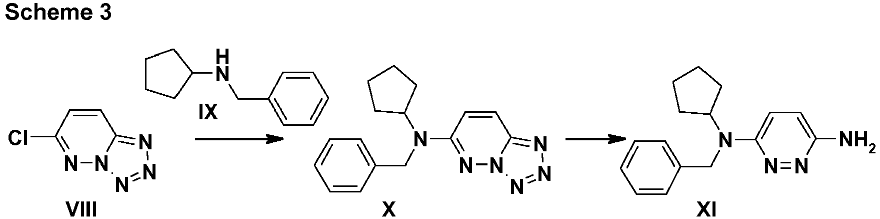

In scheme 3, the chlorotetrazolopyridazine compound of formula (VIII) is reacted with the benzylcyclopentylamine of formula (IX) at a temperature between 150 and 200°C, for example 190°C, to give the benzylcyclopentylaminotetrazolopyridazine compound of formula (X). The reaction of the latter with tributylphosphine at a temperature between 150 and 200°C, for example at 180°C, provides the aminopyridazine of formula (XI).

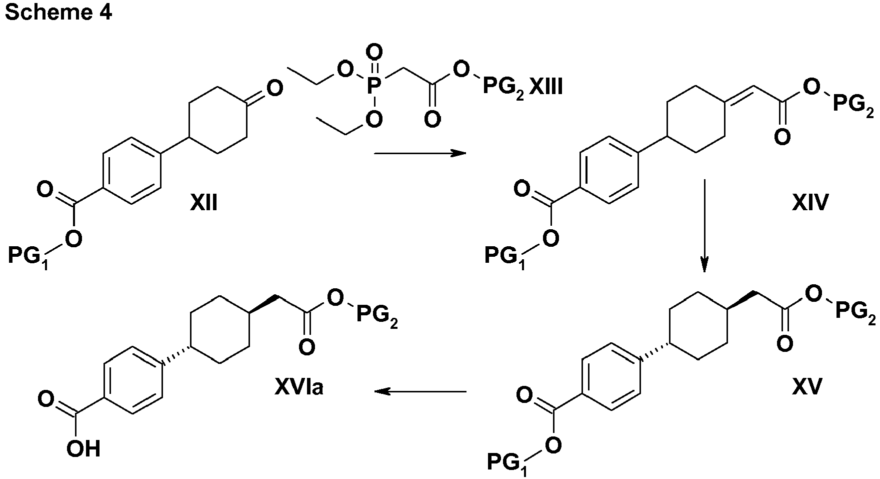

In **scheme 4,** the compound of formula (XIV) is prepared by a Horner-Wadsworth-Emmons reaction starting from ketones of formula (XII) comprising an acid function protected by a protecting group PG1 such as an ethyl group and derivatives of formulae (XIII) comprising an acid function protected by a protecting group PG2 such as a *tert*-butyl group in a polar solvent such as dimethylformamide at room temperature in the presence of a base such as sodium hydride. The compound of formula (XIV) is hydrogenated in the presence of a transition metal such as palladium in a polar solvent such as ethanol in order to give, after recrystallization, the trans compound of formula (XV). The acid of formula (XVIa) is obtained by hydrolysis of the ester of formula (XV) in the presence of lithium hydroxide in a mixture of polar solvents such as water, methanol and tetrahydrofuran.

The compounds of formula (XII) may be prepared according to a scheme described in the literature (WO 2003/099772).

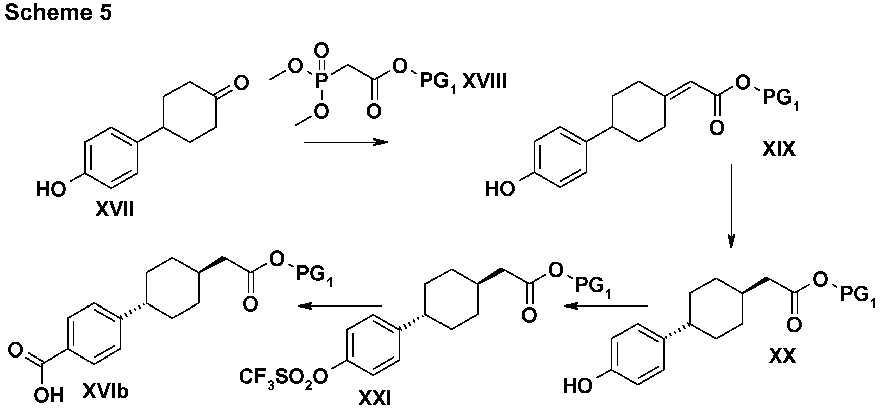

In **scheme 5,** the compound of formula (XIX) is prepared by a Horner-Wadsworth-Emmons reaction starting from the ketone of formula (XVII) and derivatives of formulae (XVIII) comprising an acid function protected by a protecting group PG1 such as a methyl group in a polar solvent such as tetrahydrofuran at room temperature in the presence of a base such as sodium hydride. The compound of formula (XIX) is hydrogenated in the presence of a transition metal such as palladium in a polar solvent such as tetrahydrofuran in order to predominantly give the trans compound of formula (XX). This is converted to the triflate of formula (XXI) using a reactant such as triflic anhydride in a polar solvent such as dichloromethane, between 0 and 25°C, in the presence of a base such as triethylamine. The acid of formula (XVIb) is obtained by hydroxycarbonylation of the triflate of formula (XXI) in a polar solvent such as dioxane, between 100 and 120°C, by methods chosen from those known to a person skilled in the art. They include, *inter alia*, the use of a carbonylation reagent such as molybdenum hexacarbonyl and of a palladium catalyst such as palladium diacetate.

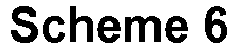

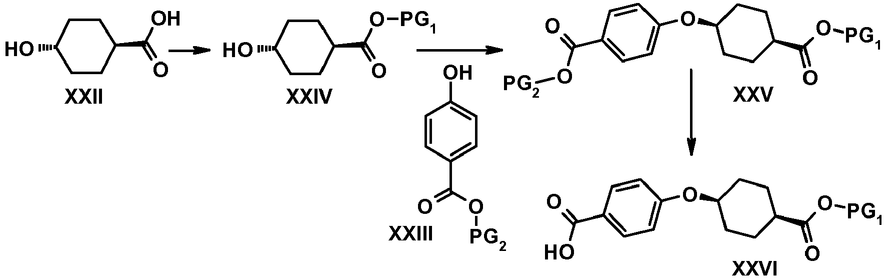

In **scheme 6,** the compound of formula (XXIV) comprising an acid function protected by a protecting group PG1, such as a methyl group, is obtained from the acid of formula (XXII) by selective reaction with a compound known to a person skilled in the art such as trimethylsilyldiazomethane in a mixture of apolar and polar solvent such as, respectively, toluene and methanol, at room temperature. The alcohol of formula (XXIV) is involved in a Mitsunobu reaction with the phenol of formula (XXIII) comprising an acid function protected by a protecting group PG2, such as a *tert*-butyl group, in a polar solvent such as tetrahydrofuran, at room temperature in order to give the ether of formula (XXV). The acid of formula (XXVI) is obtained by deprotection of the intermediate of formula (XXV) by methods chosen from those known to a person skilled in the art. These include, for an acid function protected by a protecting group PG2, such as the *tert*-butyl group, *inter alia*, the use of trifluoroacetic acid or of hydrochloric acid in polar solvents such as dichloromethane or dioxane.

Scheme 7 gives details of a synthesis of intermediates in which D represents a bond or an oxygen atom, W represents a -CH- group or a nitrogen atom, Z2 and Z3 represent methylene groups, a -CH=CH- group or are included in a cyclopropyl. These compounds will be referred to hereinbelow as compounds of formula (XXVII).

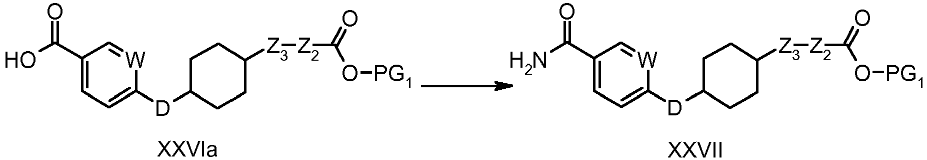

In scheme 7, the compound of formula (XXVIa) comprising an acid function protected by a protecting group PG1, such as a methyl or *tert*-butyl group, is reacted with ammonia in the presence of a coupling agent such as 1-ethyl-3-(3-dimethylaminopropyl)carbodiimide in a polar solvent such as dioxane in order to give the amide of formula (XXVII).

It should be noted tha the compounds of general formula (XXVIa) are described in the literature (WO 10/086551).

### Synthesis of molecules of interest

Scheme 8 gives details of a synthesis of the compounds of formula I in which n is equal to 1 or 0, D represents a bond or an oxygen atom, W represents a -CH- group, X1 represents a nitrogen atom or a -CH=CH- group, X2 represents an oxygen or nitrogen atom, X3 represents an oxygen atom or a nitrogen atom, R1 and R2 represent a hydrogen atom, Y represents a phenyl substituted by a substituent chosen from a halogen atom or a methoxy, Z1 and Z2 are absent and Z3 represents a methylene group. These compounds will be referred to hereinbelow as compounds of formula (XXX).

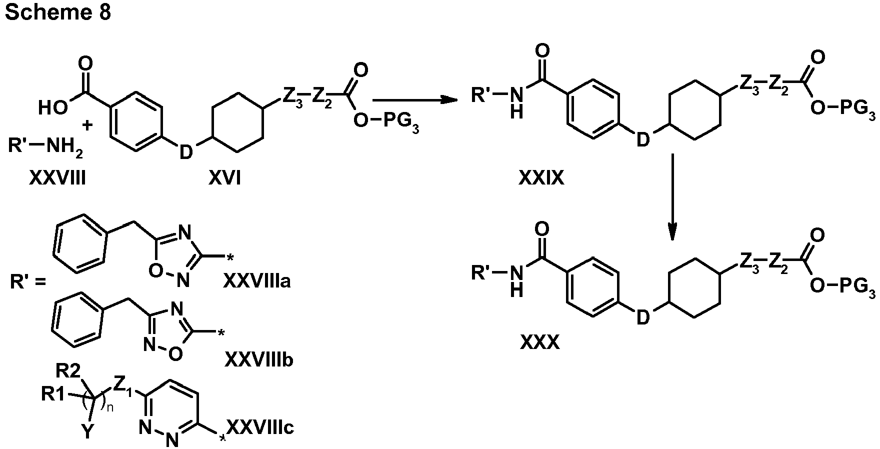

In **scheme 8,** the amines of formula (XXVIII) are involved in an amide coupling reaction with the acids of formula (XVI) comprising an acid function protected by a protecting group PG3, such as a methyl or *tert*-butyl group, in order to give the intermediates of formula (XXIX) by methods chosen from those known to a person skilled in the art. They include, *inter alia,* the use of a coupling agent such as CDI in the presence of DBU in a polar solvent such as DMF at a temperature of 100°C, or EDC in the presence of HOBt in a polar solvent such as dioxane at a temperature of 80°C in a microwave device or the acid chloride derived from the acid of formula (XVI) in the presence of a base such as triethylamine in a polar solvent such as acetonitrile. The acids of formula (XXX) are obtained after deprotection of the ester of the intermediate of formula (XXIX) by methods chosen from those known to a person skilled in the art. The esters of the intermediates of formula (XXIX) are deprotected by methods chosen from those known to a person skilled in the art. They include, *inter alia*, the use of lithium hydroxide in a mixture of polar solvents such as water, methanol and tetrahydrofuran in the case of a methyl protecting group or in the presence of an acid such as trifluoroacetic acid in the case of a *tert*-butyl protecting group.

Scheme 9 gives details of a synthesis of the compounds of formula I in which n is equal to 1, D represents a bond or an oxygen atom, W represents a -CH- group or a nitrogen atom, X1 represents a nitrogen atom, X2 represents an oxygen atom, X3 represents a nitrogen atom, R1 and R2 represent a hydrogen atom, a methyl group or may form, with the carbon atom to which they are attached, a cyclopropyl group, Y represents a phenyl or a phenoxy, Z1 is absent, Z2 is absent and Z3 represents a methylene group, Z2 and Z3 represent methylene groups, a -CH=CH- group or are included in a cyclopropyl. These compounds will be referred to hereinbelow as compounds of formula (XXXII).

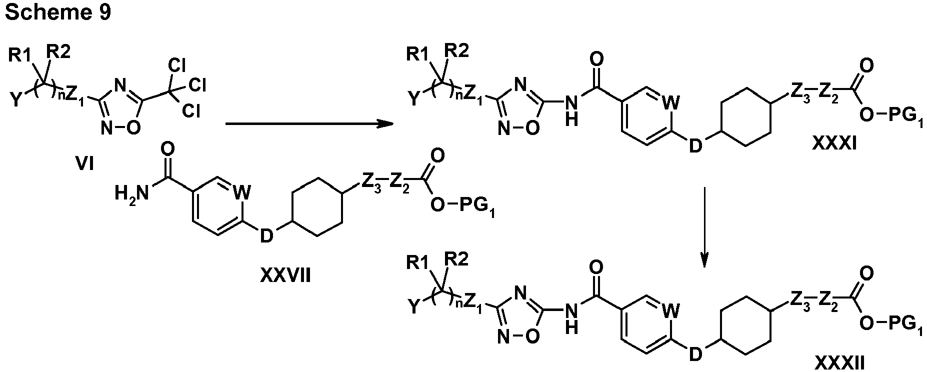

In **scheme 9,** the trichloromethyloxadiazole of formula (VI) is reacted with the amide of formula (XXVII) in the presence of a base such as sodium hydride, in a polar solvent such as tetrahydrofuran, in order to give the oxadiazoles of formula (XXXI). The latter is treated by an alkali-metal base such as lithium hydroxide in the case of a protecting group PG1 such as a methyl group or in the presence of an acid such as trifluoroacetic acid in the case of a *tert*-butyl protecting group so as to provide the corresponding carboxylic acid of formula (XXXII).

Scheme 10 gives details of a synthesis of the compounds of formula I in which n is equal to 0, D represents an oxygen atom, W represents a -CH- group, X1 represents a -CH=CH- group, X2 represents a nitrogen atom, X3 represents a nitrogen atom, R1 and R2 are absent, Y represents a (C3-C10)cycloalkyl group, Z1 represents an NH function, Z2 is absent and Z3 represents a methylene group. These compounds will be referred to hereinbelow as compounds of formula (XXXVII).

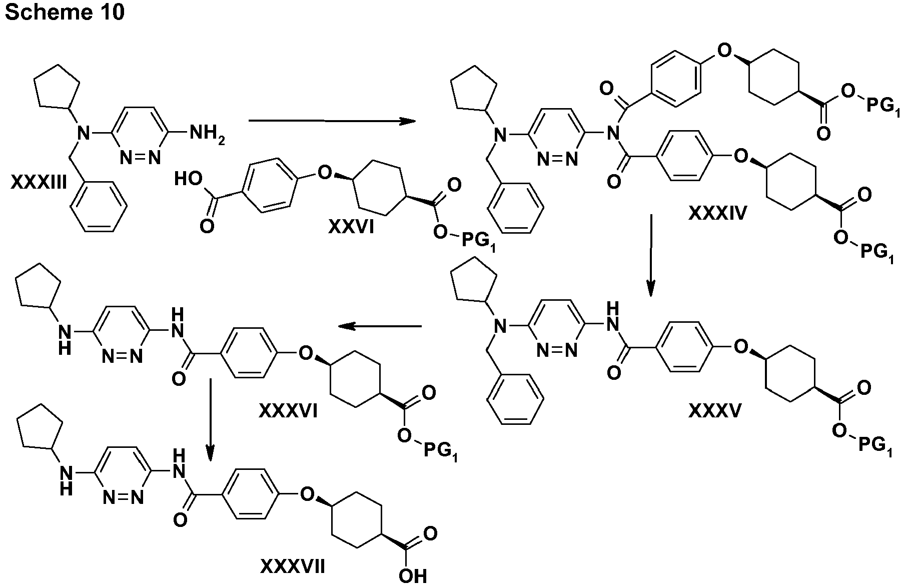

In **scheme 10,** the aminopyridazine of formula (XXXIII) is reacted with the acid chloride derived from the acid of formula (XXVI) in the presence of a base such as triethylamine, in a polar solvent such as acetonitrile, in order to give the imide of formula (XXXIV). The latter is treated with hydrazine in a basic polar solvent such as pyridine and gives the amide of formula (XXXV). This, under hydrogen pressure in the presence of a metal such as palladium and of an acid such as hydrochloric acid provides the corresponding intermediate of formula (XXXVI). The latter is treated with an alkali-metal base such as lithium hydroxide in the case of a protecting group PG1, such as a methyl group, in a mixture of polar solvents such as water and tetrahydrofuran in order to provide the corresponding carboxylic acid of formula (XXXVII).

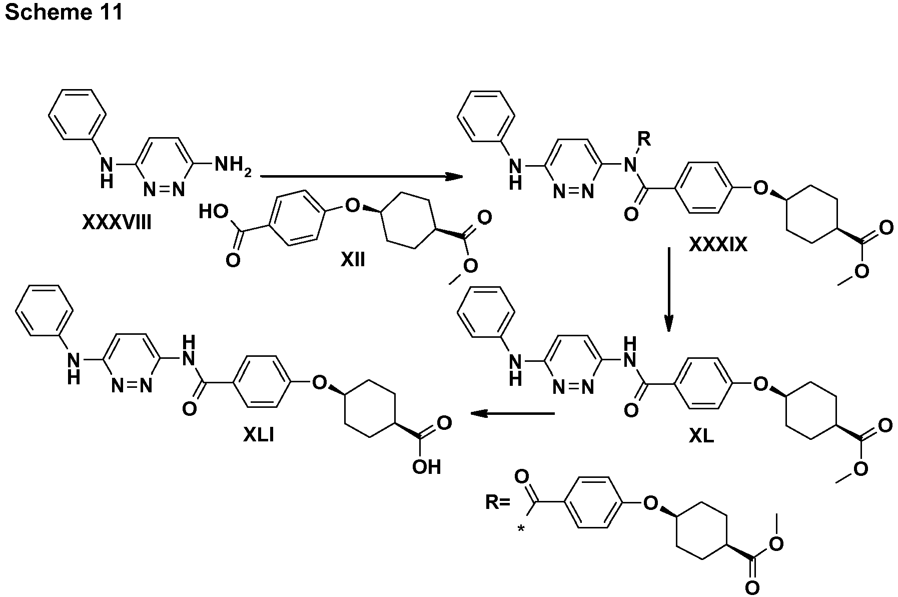

In **scheme 11,** the aminopyridazine of formula (XXXVIII) is reacted with the acid of formula (XXII) in the presence of a coupling agent (for example, 1-ethyl-3-(3-dimethylaminopropyl)carbodiimide) in a polar solvent such as dioxane, at a temperature of 80°C in a microwave device in order to give the compound of formula (XXXIX). The latter is treated with hydrazine in a basic polar solvent such as pyridine and gives the amide of formula (XL). The latter is treated with an alkali-metal base such as lithium hydroxide in a mixture of polar solvents such as water and tetrahydrofuran, in order to provide the corresponding carboxylic acid of formula (XLI).

In schemes 1 to 6, the starting compounds and the reactants, when their preparation method is not described, are commercially available or are described in the literature, or else may be prepared according to methods which are described therein or which are known to a person skilled in the art.

Another subject of the invention, according to another of its aspects, is the compounds of formulae (XXIX), (XXXI), (XXXIV), (XXXV) and (XXXVI). These compounds are of use as intermediates for the synthesis of the compounds of formula (I).

The examples that follow describe the preparation of certain compounds in accordance with the invention. These examples serve to illustrate the present invention. The numbers of the compounds given as examples refer to those given in the table hereinbelow, which illustrates the chemical structures and physical properties of a few compounds according to the invention.

The following abbreviations and empirical formulae are used:
- ACN: acetonitrile
- BSA: bovine serum albumin
- CDI: carbonyldiimidazole
- DBU: 1,8-diazabicyclo[5.4.0]undec-7-ene
- °C: degrees Celsius
- CO₂: carbon dioxide
- cm²: square centimetres
- DIEA: diisopropylethylamine
- dec.: decomposition
- DMAP: 4-dimethylaminopyridine
- DMEM: Dulbecco's Minimum Essential Medium Modified
- DMF: dimethylformamide
- DMSO: dimethylsulfoxide
- EDC: 1-ethyl-3-(3-dimethylaminopropyl)carbodiimide hydrochloride
- EDTA: ethylenediaminetetraacetic acid
- eq.: equivalent
- ESI+: electrospray ionization
- g: gram
- *g*: constant force of gravity = 9.81 m.s⁻²
- NMR: nuclear magnetic resonance
- h: hour(s)
- H₂O: water
- HOBt: hydroxybenzotriazole
- HPLC: high-performance liquid chromatography
- Hz: Hertz
- LC-MS: liquid chromatography-mass spectrometry
- M: mass
- MHz: megahertz
- mg: milligram
- mL: milliliter
- mm: millimeter
- mmol: millimoles
- N: normal
- nM: nanomolar
- PBS: phosphate-buffered saline
- m.p.: melting point
- ppm: parts per million
- psi: pounds per square inch
- FCS: fetal calf serum
- TFA: trifluoroacetic acid
- THF: tetrahydrofuran
- UPLC: ultra-high performance liquid chromatography
- UV: ultraviolet
- HIV: human immunodeficiency virus
- µCi: microcurie
- %: percentage

The radioactivity measurement is performed using a Flo One C625TR machine (Perkin-Elmer) or a MicroBeta counter (Perkin-Elmer).

The proton magnetic resonance spectra (¹H NMR), as described hereinbelow, are recorded at 400 MHz in DMSO-d₆, using the peak of DMSO-d₅ as the reference. The chemical shifts δ are expressed in parts per million (ppm). The observed signals are expressed as follows: s = singlet; d = doublet; t = triplet; m = multiplet or broad singlet.

The LC/MS column and the examples indicate the peak M+H⁺ identified by mass spectrometry. The compounds are analyzed by liquid chromatography (UV detector at 220 nm) coupled to a mass spectrometer with an electrospray ionization detector. The analytical method is detailed below:
UPLC/MS - gradient of 3 min - water/ACN/TFA
T 0 min: 98% A - T 1.6 to T 2.1 min: 100% B - T 2.5 to T 3 min: 98% A
Route A: water + 0.05% TFA, route B: ACN + 0.035% TFA
Flow rate: 1.0 mL/min - T° = 40°C - injection 2 µL
Acquity BEH C18 column (50*2.1 mm; 1.7 *µm)

### Synthesis of the intermediates

### Example 1: 5-benzyl[1.2.4]oxadiazol-3-ylamine

### 1.1 Synthesis of phenylacetyanamide

1.59 mL of phenylacetyl chloride (12 mmol, 1 eq.) are added dropwise to a solution of 2.30 g of sodium cyanamide (36 mmol, 3 eq.) in 30 mL of THF cooled to 4°C. The reaction medium is brought to reflux for 6 h. After cooling, it is diluted with ethyl acetate and washed with water. The basic aqueous phase is acidified to pH = 1 and extracted using dichloromethane. The organic phase is dried over sodium sulfate, filtered and evaporated to give phenylacetylcyanamide which is used as is in the remainder of the synthesis.

### 1.2 Synthesis of 5-benzyl[1.2.4]oxadiazol-3-ylamine

Successively added to 20 mL of ethanol are the phenylacetylcyanamide from the preceding step, 1.25 g of hydroxylamine hydrochloride (18 mmol, 1.5 eq.) and 3.4 mL of pyridine (48 mmol, 4 eq.). After reacting for 36 h, the medium is triturated in water, filtered and washed with water to give 1.12 g of 5-benzyl[1.2.4]oxadiazol-3-ylamine.
M+H⁺=176.

### Example 2: 3-benzyl[1.2.4]oxadiazol-5-ylamine

### 2.1 Synthesis of N-hydroxy-2-phenylacetamidine

Placed successively in 60 mL of ethanol are 4.60 mL of phenylacetonitrile (39.85 mmol, 1 eq.), 3.46 g of hydroxylamine hydrochloride (49.82 mmol, 1.25 eq.) and 8.33 mL of triethylamine (59.78 mmol, 1.5 eq.). The medium is brought to reflux for 3.5 h and evaporated. The residue is taken up in dichloromethane and washed with water. The organic phase is dried over sodium sulfate, filtered and evaporated. The residue is chromatographed on silica gel, eluting with a 1/1 ethyl acetate/heptane mixture. 2.9 g of N-hydroxy-2-phenylacetamidine are obtained.

### 2.2 Synthesis of 3-benzyl-5-trichloromethyl[1.2.4]oxadiazole

0.41 mL of trichloroacetyl chloride (3.66 mmol, 1.1 eq.) are added to a solution, cooled to 4°C, of 0.5 g of N-hydroxy-2-phenylacetamidine (3.33 mmol, 1 eq.) and of 1 mL of pyridine (12.36 mmol, 3.7 eq.) in 4 mL of toluene. The medium is brought to 85°C for 2.5 h, evaporated and taken up in diethyl ether. The organic phase is washed with water, dried over sodium sulfate, filtered and evaporated to give 0.83 g of 3-benzyl-5-trichloromethyl[1.2.4]oxadiazole.

### 2.3 Synthesis of 3-benzyl[1.2.4]oxadiazol-5-ylamine

0.83 g of 3-benzyl-5-trichloromethyl[1.2.4]oxadiazole (3 mmol, 1 eq.) are placed in 6 mL of methanol and 6 mL of a 7 N aqueous solution of ammonia (42 mmol, 14 eq.) are added. After 3 days, the medium is evaporated and the residue is chromatographed on silica gel, eluting with a gradient of ethyl acetate in heptane that varies from 25% to 50%. 0.270 g of 3-benzyl[1.2.4]oxadiazol-5-ylamine is obtained.

### Example 3: N-benzyl-N-cyclopentylpyridazine-3,6-diamine

### 3.1 Synthesis of benzylcyclopentyl[1.2.4]triazolo[1,5-b]pyridazin-6-ylamine

0.8 g of 6-chloro[1.2.4]triazolo[1,5-b]pyridazine (5.14 mmol, 1 eq.) and 0.9 g of benzylcyclopentylamine (5.14 mmol, 1 eq.) are placed in a sealed tube. The medium is heated at 190°C for 3 h to give 1.51 g of benzylcyclopentyl[1.2.4]triazolo[1,5-b]pyridazin-6-ylamine.
M+H⁺=295.

### 3.2 Synthesis of N-benzyl-N-cyclopentylpyridazine-3,6-diamine

1.51 g of benzylcyclopentyl[1.2.4]triazolo[1,5-b]pyridazin-6-ylamine (5.14 mmol), 2.08 g of tributylphosphine (10.28 mmol, 2 eq.) and 6 mL of acetonitrile are placed in a sealed tube. The medium is heated at 180°C for 3 h and the solvent is evaporated. The residue is taken up in 56 mL of acetic acid and 14 mL of water. The medium is brought to reflux for 3 h. After cooling, it is brought to basic pH using a saturated aqueous solution of sodium hydrogen carbonate and extracted two times using ethyl acetate. The organic phase is evaporated and the residue is chromatographed on silica gel, eluting with a dichloromethane/methanol/aqueous ammonia gradient that varies from 98/2/0.2 to 92/8/0.8 to give 0.4 g of N-benzyl-N-cyclopentylpyridazine-3,6-diamine.
M+H⁺=269.

### Example 4: 4-(4-tert-butoxycarbonylmethylcyclohexyl)benzoic acid

### 4.1 Synthesis of ethyl 4-(4-tert-butoxycarbonylmethylenecyclohexyl)benzoate

5.63 g of *tert*-butyl diethylphosphoacetate (20.3 mmol, 1 eq.) are placed in 20 mL of dimethylformamide with stirring. The solution is cooled to a temperature of 4°C by placing the solution in an ice bath, and 0.536 g of sodium hydride (22.33 mmol, 1.1 eq.) is then added portionwise. After 30 minutes, 5 g of ethyl 4-(4-oxocyclohexyl)-benzoate (20.3 mmol, 1 eq.) are added and the ice bath is removed. After stirring for 1 hour, the round-bottomed flask is placed in an ice bath to cool the reaction medium to a temperature of 4°C, and 0.049 g of sodium hydride (2.04 mmol, 0.1 eq.) is added. The ice bath is removed and, after 30 minutes, the mixture is poured into 200 mL of 1N potassium hydrogen sulfate and extracted with 300 mL of diethyl ether. The organic phase is washed 4 times with brine. The organic phase is dried over sodium sulfate and evaporated. The residue is chromatographed on silica gel, eluting with a gradient of methanol in dichloromethane ranging from 0% to 5%. 5.04 g of ethyl 4-(4-*tert-*butoxycarbonylmethylenecyclohexyl)benzoate are obtained.
M+H⁺ = 345.

### 4.2 Synthesis of ethyl 4-(4-tert-butoxycarbonylmethylcyclohexyl)benzoate

5.04 g of ethyl 4-(4-*tert*-butoxycarbonylmethylenecyclohexyl)benzoate (14.63 mmol, 1 eq.) and 15 mL of ethanol are placed in a Parr bottle. 0.31 g of 10% palladium-on-charcoal (0.29 mmol, 0.02 eq.) is added and the reaction medium is placed under 50 psi of hydrogen for 3 hours at a temperature of 25°C. The reaction medium is filtered and concentrated to give 4.31 g of ethyl 4-(4-*tert*-butoxycarbonylmethylcyclo-hexyl)benzoate.
M-isobutene⁺ = 291.

### 4.3 Synthesis of 4-(4-tert-butoxycarbonylmethylcyclohexyl)benzoic acid

3.3 g of ethyl 4-(4-*tert*-butoxycarbonylmethylcyclohexyl)benzoate (9.52 mmol, 1 eq.) are dissolved in 30 mL of a 2/1 mixture of tetrahydrofuran/methanol, then 1.60 g of lithium hydroxide hydrate (38.10 mmol, 4 eq. dissolved in 10 mL of water) are added. The reaction medium is stirred for 4 hours at room temperature. The solvents are evaporated off and an aqueous solution of SO₂ is added. The solid obtained is filtered off, washed with water and dried to give 2 g of 4-(4-*tert*-butoxycarbonylmethylcyclohexyl)benzoic acid.

### 4.4. Synthesis of trans-4-(4-tert-butoxycarbonylmethylcyclohexyl)benzoic acid

0.5 g of 4-(4-*tert*-butoxycarbonylmethylcyclohexyl)benzoic acid is recrystallized from ethyl acetate at the reflux point of the solution. After filtration and drying, 0.17 g of *trans*-4-(4-*tert*-butoxycarbonylmethylcyclohexyl)benzoic acid is obtained.

¹H NMR (400 MHz, DMSO-d6) δ ppm 12.77 (m, 1H), 7.87 (m, J = 9 Hz, 2H), 7.36 (m, J = 9 Hz, 2H), 2.54 (m, 1 H), 2.13 (d, J = 7.3 Hz, 2H), 1.87 to 1.68 (m, 5H), 1.49 (m, 2H), 1.43 (s, 9H), 1.14 (m, 2H).

### Example 5: 4-(4-methoxycarbonylmethylcyclohexyl)benzoic acid

### 5.1 Synthesis of methyl [4-(4-hydroxyphenyl)cyclohexylidene]acetate

1.69 g of 4-(4-hydroxyphenyl)cyclohexanone (77.22 mmol, 1 eq.) are placed in 100 mL of THF in a 250 mL round-bottomed flask under nitrogen. The solution is cooled to 4°C on an ice bath and 3 g of 60% sodium hydride (75.04 mmol, 0.97 eq.) are added portionwise. 16.88 g of methyl dimethylphosphoacetate (92.67 mmol, 1.2 eq.) are placed in 100 mL of THF in another 500 mL round-bottomed flask under nitrogen. This second solution is cooled on an ice bath and 4.41 g of 60% sodium hydride (110.30 mmol, 1.42 eq.) are added portionwise. The ice baths are removed and the media are stirred at room temperature for 30 minutes. The 4-(4-hydroxyphenyl)cyclohexanone solution is added to the methyl dimethylphosphoacetate solution. The reaction medium is stirred for 18 h. 100 mL of a saturated aqueous ammonium chloride solution is added and the reaction medium is extracted three times with ethyl acetate. The organic phase is washed with water, dried over magnesium sulfate, filtered and evaporated to give 17.16 g of methyl [4-(4-hydroxyphenyl)cyclohexylidene]acetate.
M+H⁺ = 247.

### 5.2 Synthesis of methyl [4-(4-hydroxyphenyl)cyclohexyl]acetate

17.16 g of methyl [4-(4-hydroxyphenyl)cyclohexylidene]acetate (69.67 mmol, 1 eq.) are placed in 130 mL of THF in a Parr bottle under nitrogen. 2.96 g of 5% palladium-on-charcoal (1.39 mmol, 0.02 eq.) is added and the reaction medium is placed under 50 psi of hydrogen for 3.5 h at 30°C. The reaction medium is filtered and concentrated to give 16.63 g of methyl [4-(4-hydroxyphenyl)cyclohexyl]acetate in a trans/cis ratio of 70/30.
M+H⁺ = 249.

### 5.3 Synthesis of methyl [4-(4-trifluoromethanesulfonyloxyphenyl)cyclohexyl]-acetate

16.63 g of methyl [4-(4-hydroxyphenyl)cyclohexyl]acetate (66.96 mmol, 1 eq.) are placed in 200 mL of dichloromethane. The solution is cooled on an ice bath, and 14 mL of diisopropylethylamine (80.36 mmol, 1.2 eq.) and 13.3 mL of triflic anhydride (80.36 mmol, 1.2 eq.) are added successively and dropwise while not exceeding 6°C. The ice bath is removed and the reaction medium is stirred for 18 h. The medium is poured into ice-water and extracted with dichloromethane. The organic phase is washed with a 1 N aqueous solution of sodium hydroxide, washed with brine, dried over magnesium sulfate, filtered and evaporated. The residue is chromatographed on silica gel, eluting with a gradient of ethyl acetate in heptane ranging from 10% to 50%. 17.7 g of methyl [4-(4-trifluoromethanesulfonyloxyphenyl)cyclohexyl]acetate are obtained in a trans/cis ratio of 70/30.
M+H⁺ = 381.

### 5.4 Synthesis of 4-(4-methoxycarbonylmethylcyclohexyl)benzoic acid

0.4 g of methyl [4-(4-trifluoromethanesulfonyloxyphenyl)cyclohexyl]acetate (1.05 mmol, 1 eq.) is placed, respectively, in 3 mL of dioxane in one 20 mL microwave tube. 0.142 g of molybdenum hexacarbonyl (0.53 mmol, 0.5 eq.), 0.024 g of palladium (II) acetate (0.11 mmol, 0.1 eq.), 0.058 g of 1,1'-bis(diphenylphosphino)ferrocene (0.11 mmol, 0.1 eq.), 0.257 g of 4-dimethylaminopyridine (2.1 mmol, 2 eq.), 0.42 mL of diisopropylethylamine (2.42 mmol, 2.3 eq.) and 0.38 mL of water (21.03 mmol, 20 eq.) are successively added. The tube is heated at 120°C for 10 minutes in a Biotage microwave machine. The reaction medium is filtered. The filtrate is washed with a saturated sodium carbonate solution and diluted with diethyl ether. After separation of the phases by settling, the aqueous phase is acidified with a 5N hydrochloric acid solution and extracted twice with dichloromethane. The organic phase is dried over sodium sulphate and concentrated to dryness to give 0.155 g of 4-(4-methoxycarbonylmethylcyclohexyl)benzoic acid in a trans/cis ratio of 70/30.

### Example 6: cis-4-(4-methoxycarbonylcyclohexyloxy)benzoic acid

### 6.1 Synthesis of methyl trans-4-hydroxycyclohexanecarboxylate

1 g of 4-hydroxycyclohexanecarboxylic acid (6.94 mmol, 1 eq.) is placed in 50 mL of a 4/1 mixture of toluene/methanol. 5.55 mL of a 2 N solution of trimethylsilyldiazomethane in hexane (11.10 mmol, 1.6 eq.) is poured dropwise into the reaction medium with stirring. After 16 hours, the solvents are evaporated to give 1.3 g of methyl *trans*-4-hydroxycyclohexanecarboxylate.

### 6.2 Synthesis of tert-butyl cis-4-(4-methoxycarbonylcyclohexyloxy)benzoate

1.1 g of methyl *trans*-4-hydroxycyclohexanecarboxylate (6.95 mmol, 1.35 eq.), 1 g of *tert*-butyl 4-hydroxybenzoate (5.15 mmol, 1 eq.) and 2.03 g of triphenylphosphine (7.72 mmol, 1.5 eq.) are placed in 10 mL of tetrahydrofuran at room temperature. 1.5 mL of diisopropyl azodicarboxylate (7.72 mmol, 1.5 eq.) are added dropwise to the reaction medium with stirring. After 18 hours at room temperature, the medium is concentrated to dryness and taken up in diethyl ether. The triphenylphosphine oxide is filtered off. The organic phase is washed with a sodium hydroxide solution, dried over sodium sulfate, filtered and evaporated to give a residue. This residue is chromatographed on silica gel, eluting with a gradient of ethyl acetate in heptane ranging from 0% to 50%. 1.23 g of *tert*-butyl *cis*-4-(4-methoxycarbonylcyclohexyloxy)-benzoate are obtained.

### 6.3 Synthesis of cis-4-(4-methoxycarbonylcyclohexyloxy)benzoic acid

0.6 g of *tert*-butyl *cis*-4-(4-methoxycarbonylcyclohexyloxy)benzoate (1.79 mmol, 1 eq.) are placed in 2.5 mL of dichloromethane. The reaction medium is cooled to a temperature of 4°C, with stirring, in an ice bath. 1 mL of trifluoroacetic acid (13.46 mmol, 7.5 eq.) is added and the ice bath is removed. After stirring for 5 hours at room temperature, the medium is concentrated, taken up in diethyl ether, drained and filtered to give 0.30 g of *cis* -4-(4-methoxycarbonylcyclohexyloxy)benzoic acid.
M+H⁺ = 279.

### Example 7: Methyl cis-4-(4-carbamoylphenoxy)cyclohexanecarboxylate

### 7.1 Synthesis of methyl cis-4-(4-carbamoylphenoxy)cyclohexanecarboxylate

3 g of *cis*-4-(4-methoxycarbonylcyclohexyloxy)benzoic acid (10.78 mmol, 1 eq.) are dissolved in 40 mL of dioxane. 1.46 g of HOBt (10.78 mmol, 1 eq.), 4.13 g of EDC (21.56 mmol, 2 eq.) and 108 mL of a 0.5 N solution of aqueous ammonia (53.90 mmol, 5 eq.) are successively added to the dioxane. After stirring for 18 h, the reaction medium is evaporated and taken up in dichloromethane. The organic phase is evaporated, washed using a 1 N solution of hydrochloric acid, washed twice with brine, dried over sodium sulfate, filtered and evaporated to give 2.99 g of methyl *cis*-4-(4-carbamoylphenoxy)cyclohexanecarboxylate.
M+H⁺=278.

### Example 8: tert-butyl cis-(5-carbamoylpyridin-2-yloxy)cyclohexanecarboxylate

This compound is obtained from the carboxylic acid according to preparation 7.1. M+H⁺ = 321.

### Example 9: tert-butyl trans-(E)-3-[4-(4-carbamoylphenyl)cyclohexyl]acrylate

This compound is obtained from the carboxylic acid according to preparation 7.1.

### Example 10: tert-butyl trans-3-[4-(4-carbamoylphenyl)cyclohexyl]propionate

This compound is obtained from the carboxylic acid according to preparation 7.1.

### Example 11: tert-butyl trans-2-[4-(4-carbamoylphenyl)cyclohexyl]cyclopropane carboxylate

This compound is obtained from the carboxylic acid according to preparation 7.1.

### Synthesis of molecules of interest

### Example 12: trans-{4-[4-(5-benzyl[1.2.4]oxadiazol-3-ylcarbamoyl)phenyl]cyclohexyl}acetic acid (compound No. 1)

### 12.1 Synthesis of tert-butyl trans-{4-[4-(5-benzyl[1.2.4]oxadiazol-3-ylcarbamoyl)phenyl]-cyclohexyl}acetate

Added to 6 mL of dimethylformamide are 0.318 g of *trans*-4-(4-*tert-*butoxycarbonylmethylcyclohexyl)benzoic acid (1 mmol, 1 eq.) and 0.292 g of CDI. After stirring for 1 h, 0.350 g of 5-benzyl[1.2.4]oxadiazol-3-ylamine (2 mmol, 2 eq.) and 0.30 mL of DBU (2 mmol, 2 eq.) are added. The medium is brought to 100°C for 24 h. After cooling, the medium is diluted with ethyl acetate and washed with water. The organic phase is dried over sodium sulfate, filtered and evaporated. The residue is chromatographed on silica gel, eluting with a 1/3 ethyl acetate/heptane mixture. 0.060 g of *tert*-butyl *trans*-{4-[4-(5-benzyl[1.2.4]oxadiazol-3-ylcarbamoyl)phenyl]cyclohexyl}acetate is obtained.

### 12.2 Synthesis of trans-{4-[4-(5-benzyl[1.2.4]oxadiazol-3-ylcarbamoyl)phenyl]cyclohexyl}acetic acid

0.060 g of *tert*-butyl *trans*-{4-[4-(5-benzyl[1.2.4]oxadiazol-3-ylcarbamoyl)phenyl]-cyclohexyl}acetate are dissolved in 3 mL of dichloromethane and 0.5 mL of trifluoroacetic acid is added. After 1 h, the medium is evaporated and triturated in ethyl acetate to give 0.015 mg of *trans*-{4-[4-(5-benzyl[1.2.4]oxadiazol-3-ylcarbamoyl)phenyl]cyclohexyl}acetic acid.
M+H⁺ = 420.
¹H NMR (400 MHz, DMSO-d6) δ ppm 1.12 (m, 2 H); 1.50 (m, 2 H); 1.74 (m, 1 H); 1.83 (m, 4 H); 2.15 (d, J=6.8 Hz, 2 H); 2.59 (m, 1 H); 3.75 (s, 2 H); 7.21 to 7.40 (m, 5 H); 7.50 (broad d, J=8.9 Hz, 2 H); 7.97 (broad d, J=8.9 Hz, 2 H); 11.43 (broad s, 1 H); 12.02 (broad unresolved m, 1 H).

### Example 13: trans-{4-[4-(3-benzyl[1.2.4]oxadiazol-5-ylcarbamoyl)phenyl]cyclohexyl}acetic acid (compound No. 2)

### 13.1 Synthesis of tert-butyl trans-{4-[4-(3-benzyl[1.2.4]oxadiazol-5-ylcarbamoyl)phenyl]-cyclohexyl}acetate

This compound is obtained according to preparation 12.1 starting from 3-benzyl[1.2.4]oxadiazol-5-ylamine and *trans*-4-(4-*tert*-butoxycarbonylmethylcyclohexyl)benzoic acid.

### 13.2 Synthesis of trans-{4-[4-(3-benzyl[1.2.4]oxadiazol-5-ylcarbamoyl)phenyl]cyclohexyl}acetic acid

0.190 g of *tert*-butyl *trans*-{4-[4-(3-benzyl[1.2.4]oxadiazol-5-ylcarbamoyl)-phenyl]cyclohexyl}acetate are dissolved in 5 mL of dichloromethane and 2 mL of trifluoroacetic acid are added. After 1 h, the medium is evaporated and triturated in ethanol and washed with water to give 0.140 mg of *trans*-{4-[4-(3-benzyl[1.2.4]oxadiazol-5-ylcarbamoyl)phenyl]cyclohexyl}acetic acid.
M+H⁺ = 420.
¹H NMR (400 MHz, DMSO-d6) δ ppm 1.12 (m, 2 H); 1.50 (m, 2 H); 1.73 (m, 1 H); 1.81 (m, 4 H); 2.15 (d, J=7.0 Hz, 2 H); 2.55 (m, 1 H); 4.03 (s, 2 H); 7.23 to 7.36 (m, 5 H); 7.39 (broad d, J=8.9 Hz, 2 H); 7.91 (broad d, J=8.9 Hz, 2 H); 11.99 (broad unresolved m, 1 H); 12.32 (broad unresolved m, 1 H).

### Example 14: {4-[4-(6-benzylpyridazin-3-ylcarbamoyl)phenyl]cyclohexyl}acetic acid (compound No. 3)

### 14.1 Synthesis of methyl {4-[4-(6-benzylpyridazin-3-ylcarbamoyl)phenyl]cyclohexyl}-acetate

0.093 g of 6-benzylpyridazin-3-ylamine (0.5 mmol, 1 eq.), 0.069 g of 4-(4-methoxycarbonylmethylcyclohexyl)benzoic acid (0.25 mmol, 0.5 eq.), 0.096 g of EDC (0.5 mmol, 1 eq.) and 0.034 g of HOBt (0.25 mmol, 0.5 eq.) are placed successively into 3 mL of DMF. After stirring for 6 days, the medium is diluted with ethyl acetate. The organic phae is washed four times with water, dried over sodium sulfate, filtered and evaporated. The residue is chromatographed on silica gel, eluting with a gradient of methanol in dichloromethane ranging from 1% to 2%. 0.043 g of *tert*-butyl {4-[4-(6-benzylpyridazin-3-ylcarbamoyl)phenyl]cyclohexyl}acetate is obtained in a trans/cis ratio of 70/30.

### 14.2 Synthesis of {4-[4-(6-benzylpyridazin-3-ylcarbamoyl)phenyl]cyclohexyl}acetic acid

0.043 g of *tert*-butyl {4-[4-(6-benzylpyridazin-3-ylcarbamoyl)phenyl]cyclohexyl}-acetate (0.1 mmol, 1 eq.) is dissolved in 3 mL of a 2/1 mixture of THF/MeOH. 0.012 g of lithium hydroxide monohydrate (0.19 mmol, 2 eq.) dissolved in 1 mL of water is added. After stirring for 18 h, the medium is partially evaporated and diluted with dichloromethane. The organic phase is washed using a 1 N solution of hydrochloric acid, dried over sodium sulfate, filtered and evaporated. The residue is triturated in an MeOH/water mixture to give 0.035 g of *trans*-{4-[4-(6-benzylpyridazin-3-ylcarbamoyl)-phenyl]cyclohexyl}acetic acid in a trans/cis ratio of 70/30.
M+H⁺ = 430.
¹H NMR (400 MHz, DMSO-d6) δ ppm 1.13 (m, 1.4 H); 1.50 (m, 1.4 H); 1.58 to 1.89 (m, 5.2 H) 1.73 (m, 0.7 H); 2.15 (d, J=7.0 Hz, 1.4 H); 2.191 (m, 0.3 H); 2.38 (d, J=7.5 Hz, 0.6 H); 2.56 (m, 0.7 H); 2.64 (m, 0.3 H); 4.27 (s, 2 H); 7.18 to 7.35 (m, 5 H); 7.38 (broad d, J=8.9 Hz, 1.4 H); 7.43 (broad d, J=8.9 Hz, 0.6 H); 7.59 (d, J=9.0 Hz, 1 H); 7.99 (m, 2 H); 8.28 (d, J=9.0 Hz, 1 H); 11.25 (broad s, 1 H); 12.02 (broad unresolved m, 1 H).

### Example 15: cis-4-[4-(3-benzyl[1.2.4]oxadiazol-5-ylcarbamoyl)phenoxy]cyclohexanecarboxylic acid (compound No. 4)

### 15.1 Synthesis of methyl cis-4-[4-(3-benzyl[1.2.4]oxadiazol-5-ylcarbamoyl)phenoxy]-cyclohexanecarboxylate

0.227 g of methyl *cis*-4-(4-carbamoylphenoxy)cyclohexanecarboxylate (1 mmol, 1 eq.) is dissolved in 5 mL of THF. 0.024 g of sodium hydride (1 mmol, 1 eq.) is added and the stirring is continued for 10 minutes. 0.555 g of 3-benzyl-5-trichloromethyl-[1.2.4]oxadiazole (2 mmol, 2 eq.) in solution in 2 mL of THF is added dropwise. After 18 h, the medium is diluted with dichloromethane and washed with water. The organic phase is dried over sodium sulfate, filtered and evaporated to give a residue which is chromatographed on silica gel, eluting with a gradient of ethyl acetate in heptane that varies from 25% to 50%. 0.194 g of methyl *cis*-4-[4-(3-benzyl[1.2.4]oxadiazol-5-ylcarbamoyl)phenoxy]cyclohexanecarboxylate is obtained.
M+H⁺=436.

### 15.2 Synthesis of cis-4-[4-(3-benzyl[1.2.4]oxadiazol-5-ylcarbamoyl)phenoxy]cyclohexanecarboxylic acid

0.194 g of methyl *cis*-4-[4-(3-benzyl[1.2.4]oxadiazol-5-ylcarbamoyl)phenoxy]cyclohexane carboxylate (0.45 mmol, 1 eq.) is dissolved in 2 mL of THF. 0.056 g of lithium hydroxide monohydrate (1.34 mmol, 3 eq.) dissolved in 1 mL of water is added. After stirring for 18 h, the medium is acidified using a 1 N solution of hydrochloric acid to an acid pH and evaporated. The residue is triturated in ethanol to give 0.095 mg of *cis*-4-[4-(3-benzyl[1.2.4]oxadiazol-5-ylcarbamoyl)phenoxy]cyclohexanecarboxylic acid.
M+H⁺ = 422.
¹H NMR (400 MHz, DMSO-d6) δ ppm 1.56 to 1.89 (m, 8 H); 2.39 (m, 1 H); 4.03 (s, 2 H); 4.62 to 4.74 (m, 1 H); 7.06 (broad d, J=8.9 Hz, 2 H); 7.21 to 7.40 (m, 5 H); 7.96 (broad d, J=8.9 Hz, 2 H); 12.10 (broad s, 1 H); 12.21 (broad s, 1 H).

### Example 16: (E)-3-{4-[4-(3-benzyl[1.2.4]oxadiazol-5-ylcarbamoyl)phenyl]cyclohexyl}acrylic acid (compound No. 3c)

### 16.1 tert-butyl trans-(E)-3-{4-[4-(3-benzyl[1.2.4]oxadiazol-5-ylcarbamoyl)phenyl]cyclohexyl}acrylate

The compound is obtained from *tert*-butyl *trans*-(E)-3-[4-(4-carbamoylphenyl)cyclohexyl]acrylate and 3-benzyl[1.2.4]oxadiazol-5-ylamine according to preparation 15.1.
M+H⁺ = 488

### 16.2 trans-(E)-3-{4-[4-(3-benzyl[1.2.4]oxadiazol-5-ylcarbamoyl)phenyl]cyclohexyl}-acrylic acid

0.257 g of *tert*-butyl *trans*-(E)-3-]4-[4-(3-benzyl[1.2.4]oxadiazol-5-ylcarbamoyl)-phenyl]cyclohexyl}acrylate are dissolved in 5 mL of dichloromethane and 0.045 mL (6.33 mmol, 12 eq.) of trifluoroacetic acid is added. After 18 h, the medium is evaporated, triturated in diethyl ether and washed with water to give a residue which is chromatographed on a C18-grafted silica column. The elution is carried out by eluting with a gradient of water containing 0.1% of TFA in a water/acetonitrile (90/10) mixture containing 0.1% of TFA (0.1%) varying from 0 to 90% to give 44 mg of *trans*-(E)-3-{4-[4-(3-benzyl[1.2.4]oxadiazol-5-ylcarbamoyl)phenyl]cyclohexyl}acrylic acid.
M+H⁺ = 432
¹H NMR (400 MHz, DMSO-d₆) δ ppm 1.31 (m, 2 H); 1.55 (m, 2 H); 1.86 (m, 4 H); 2.24 (m, 1 H); 2.59 (m, 1 H); 4.04 (s, 2 H); 5.75 (dd, J=1.5 and 15.8 Hz, 1 H); 6.82 (dd, J=6.8 and 15.8 Hz, 1 H); 7.22 to 7.38 (m, 5 H); 7.40 (d, J=8.5 Hz, 2 H); 7.92 (d, J=8.5 Hz, 2 H); 12.14 (broad s, 1 H); 12.32 (broad s, 1 H).

### Example 17: trans-3-{4-[4-(3-benzyl[1.2.4]oxadiazol-5-ylcarbamoyl)phenyl]cyclohexyl}propionic acid (compound No. 3d)

### 17.1 tert-butyl trans-3-{4-[4-(3-benzyl[1.2.4]oxadiazol-5-ylcarbamoyl)phenyl]cyclohexyl}propionate

The compound is obtained from *tert*-butyl *trans*-3-[4-(4-carbamoylphenyl)cyclohexyl]propionate and 3-benzyl[1.2.4]oxadiazol-5-ylamine according to preparation 15.1.
M+H⁺ = 490

### 17.2 trans-3-{4-[4-(3-benzyl[1.2.4]oxadiazol-5-ylcarbamoyl)phenyl]cyclohexyl}propionic acid

0.195 g of *tert*-butyl *trans*-3-{4-[4-(3-benzyl[1.2.4]oxadiazol-5-ylcarbamoyl)-phenyl]cyclohexyl}propionate are dissolved in 4 mL of dichloromethane and 0.03 mL (4.8 mmol, 12 eq.) of trifluoroacetic acid is added. After 18 h, the medium is evaporated, triturated in diethyl ether and washed with water to give 0.070 mg of trans-3-{4-[4-(3-benzyl[1.2.4]oxadiazol-5-ylcarbamoyl)phenyl]cyclohexyl}propionic acid.
M+H⁺ = 434
¹H NMR (400 MHz, DMSO-d₆) δ ppm 1.05 (m, 2 H); 1.30 (m, 1 H); 1.39 to 1.52 (m, 4 H); 1.81 (m, 4 H); 2.25 (t, J=7.7 Hz, 2 H); 2.57 (m, 1 H); 4.03 (s, 2 H); 7.20 to 7.36 (m, 5 H); 7.38 (d, J=8.3 Hz, 2 H); 7.91 (d, J=8.3 Hz, 2 H); 11.96 (broad s, 1 H); 12.31 (broad s, 1 H).

### Example 18: trans-2-{4-[4-(3-benzyl[1.2.4]oxadiazol-5-ylcarbamoyl)phenyl]cyclohexyl}cyclopropanecarboxylic acid (compound No. 3b)

### 18.1 tert-butyl trans-2-{4-[4-(3-benzyl[1.2.4]oxadiazol-5-ylcarbamoyl)phenyl]cyclohexyl}cyclopropane carboxylate

The compound is obtained from *tert*-butyl *trans*-2-[4-(4-carbamoylphenyl)cyclohexyl]-cyclopropanecarboxylate and 3-benzyl[1.2.4]oxadiazol-5-ylamine according to preparation 15.1.
M+H⁺ = 502

### 18.2 trans-2-{4-[4-(3-benzyl[1.2.4]oxadiazol-5-ylcarbamoyl)phenyl]cyclohexyl}cyclopropanecarboxylic acid

The compound is obtained from *tert*-butyl *trans*-2-{4-[4-(3-benzyl[1.2.4]oxadiazol-5-ylcarbamoyl)phenyl]cyclohexyl}cyclopropanecarboxylate according to preparation 16.2.
M+H⁺ = 446
¹H NMR (400 MHz, DMSO-d₆) δ ppm 0.66 to 0.88 (m, 2 H); 0.93 (m, 1 H); 1.09 (m, 1 H); 1.24 (m, 2 H); 1.32 to 1.51 (m, 3 H); 1.75 to 1.93 (m, 4 H); 2.56 (m, 1 H); 4.04 (s, 2 H); 7.20 to 7.42 (m, 7 H); 7.91 (d, J=8.3 Hz, 2 H); 11.92 (broad unresolved m, 1 H); 12.31 (s, 1 H).

### Example 19: cis-4-[5-(3-benzyl[1.2.4]oxadiazol-5-ylcarbamoyl)pyridin-2-yloxy]-cyclohexanecarboxylic acid (compound No. 10)

### 19.1 tert-butyl cis-4-[5-(3-benzyl[1.2.4]oxadiazol-5-ylcarbamoyl)pyridin-2-yloxy]cyclohexanecarboxylate

The compound is obtained from *tert*-butyl cis-(5-carbamoylpyridin-2-yloxy)cyclohexanecarboxylate and 3-benzyl[1.2.4]oxadiazol-5-ylamine according to preparation 15.1.
M+H⁺ = 479

### 19.2 cis-4-[5-(3-benzy[1.2.4]oxadiazol-5-ylcarbamoyl)pyridin-2-yloxy]cyclohexane-carboxylic acid

The compound is obtained from *tert*-butyl *cis*-4-[5-(3-benzyl[1.2.4]oxadiazol-5-ylcarbamoyl)pyridin-2-yloxy]cyclohexanecarboxylate according to preparation 13.2.
M+H⁺ = 423
¹H NMR (400 MHz, DMSO-d₆) δ ppm 1.49 to 1.97 (m, 8 H); 2.39 (m, 1 H); 4.03 (s, 2 H); 5.25 (m, 1 H); 6.91 (d, J=8.8 Hz, 1 H); 7.17 to 7.43 (m, 5 H); 8.21 (dd, J=2.5 and 8.8 Hz, 1 H); 8.77 (d, J=2.5 Hz, 1 H); 12.10 (broad s, 1 H); 12.41 (broad s, 1 H).

### Example 20: cis 4-[4-(6-cyclopentylaminopyridazin-3-ylcarbamoyl)phenoxy]-cyclohexanecarboxylic acid (compound No. 9)

### 20.1 Synthesis of methyl cis-4-{4-[cis-4-(4-methoxycarbonylcyclohexyloxy)benzoyl(6-cyclopentylaminopyridazin-3-yl)aminocarbonyl]phenoxy}cyclohexanecarboxylate

0.4 g of *cis*-4-(4-methoxycarbonylcyclohexyloxy)benzoic acid (1.44 mmol, 1 eq.) is placed into 5 mL of dichloromethane. Two drops of DMF and 0.24 mL of oxalyl chloride (1.87 mmol, 1.3 eq.) are successively added. After stirring for 1.5 h, the medium is evaporated to form *cis*-4-(4-methoxycarbonylcyclohexyloxy)benzoyl chloride. Placed into a round-bottomed flask are 0.4 g of N-benzyl-N-cyclopentyl-pyridazine-3,6-diamine (1.49 mmol, 1.04 eq.) and 0.26 mL of triethylamine (1.87 mmol, 1.3 eq.) in 5 mL of acetonitrile and *cis*-4-(4-methoxycarbonylcyclohexyloxy)benzoyl chloride dissolved in 2 mL of acetonitrile is added dropwise. After 2.5 h, the medium is diluted with dichloromethane, washed with a saturated solution of sodium hydrogencarbonate, washed twice with water, washed with brine, dried over sodium sulfate, filtered and evaporated. The residue is chromatographed on silica gel, eluting with a gradient of ethyl acetate in heptane that varies from 10% to 50%. 0.37 g of methyl *cis*-4-{4-[cis-4-(4-methoxycarbonylcyclohexyloxy)benzoyl(6-cyclopentylamino-pyridazin-3-yl)aminocarbonyl]phenoxy}cyclohexanecarboxylate is obtained.
M+H⁺ = 789.

### 20.2 Synthesis of methyl cis-4-{4-[6-(benzylcyclopentylamino)pyridazin-3-ylcarbamoyl]-phenoxy}cyclohexanecarboxylate

0.32 g of methyl *cis*-4-{4-[cis-4-(4-methoxycarbonylcyclohexyloxy)benzoyl-(6-cyclopentylaminopyridazin-3-yl)aminocarbonyl]phenoxy}cyclohexanecarboxylate (0.41 mmol, 1 eq.) and 0.03 mL of hydrazine hydrate (0.62 mmol, 1.52 eq.) are placed in 2 mL of pyridine. After stirring for 18 h, the medium is evaporated and diluted with dichloromethane. The organic phase is washed with a saturated solution of sodium hydrogencarbonate, washed with brine, dried over sodium sulfate, filtered and evaporated. The residue is chromatographed on silica gel, eluting with a gradient of methanol in dichloromethane that varies from 1% to 5%. 0.2 g of methyl *cis*-4-{4-[6-(benzylcyclopentylamino)pyridazin-3-ylcarbamoyl]phenoxy}cyclohexanecarboxylate is obtained.
M+H⁺ = 529.

### 20.3 Synthesis of methyl cis-4-[4-(6-cyclopentylaminopyridazin-3-ylcarbamoyl)-phenoxylcyclohexanecarboxylate

0.220 g of methyl *cis*-4-{4-[6-(benzylcyclopentylamino)pyridazin-3-ylcarbamoyl]phenoxy}cyclohexanecarboxylate (0.42 mmol, 1 eq.) and 20 mL of methanol are placed in a Parr bottle. 0.07 g of 10% palladium-on-charcoal (0.07 mmol, 0.16 eq.) and 0.05 mL of a 4N solution of hydrochloric acid in dioxane are added. The reaction medium is put under 50 psi of hydrogen for 10 h at a temperature of 25°C. The reaction medium is filtered and concentrated. The residue is chromatographed on silica gel, eluting with a gradient of methanol in dichloromethane that varies from 2% to 5%. 0.15 g of methyl *cis*-4-[4-(6-cyclopentylaminopyridazin-3-ylcarbamoyl)phenoxy]cyclohexanecarboxylate is obtained.
M+H⁺ = 439.

### 20.4 Synthesis of cis-4-[4-(6-cyclopentylaminopyridazin-3-ylcarbamoyl)phenoxy]cyclohexanecarboxylic acid

0.135 g of methyl *cis*-4-[4-(6-cyclopentylaminopyridazin-3-ylcarbamoyl)-phenoxy]cyclohexanecarboxylate (0.31 mmol, 1 eq.) is placed in 4 mL of tetrahydrofuran and 0.039 g of lithium hydroxide monohydrate (0.92 mmol, 3 eq.) dissolved in 2 mL of water is added. After stirring for 18 h, the medium is acidified using a 1 N solution of hydrochloric acid and extracted three times with dichloromethane. The organic phase is dried over sodium sulfate, filtered and evaporated. The residue is triturated successively in ethyl acetate and diethyl ether to give 0.051 g of *cis*-4-[4-(6-cyclopentylaminopyridazin-3-ylcarbamoyl)phenoxy]cyclohexanecarboxylic acid.
M+H⁺ = 425.
¹H NMR (400 MHz, DMSO-d₆) δ ppm 1.43 to 2.04 (m, 16 H); 2.40 (m, 1 H); 4.18 (m, 1 H); 4.69 (m, 1 H); 7.06 (broad d, J=8.9 Hz, 2 H); 7.12 (broad d, J=7.5 Hz, 1 H); 7.61 (very broad unresolved m, 1 H); 7.92 to 8.02 (m, 3 H); 10.79 (broad s, 1 H); 12.10 (broad unresolved m, 1 H).

### Example 21: cis-4-[4-(6-phenylaminopyridazin-3-ylcarbamoyl)phenoxy]cyclohexanecarboxylic acid (compound No. 11)

### 21.1 Synthesis of methyl cis-4-{4-[cis-4-(4-methoxycarbonylcyclohexyloxy)benzoyl-(6-phenylaminopyridazin-3-yl)aminocarbonyl]phenoxy}cyclohexanecarboxylate

0.053 g of 6-phenylaminopyridazin-3-ylamine (0.28 mmol, 1 eq.), 0.079 g of *cis-*4-(4-methoxycarbonylcyclohexyloxy)benzoic acid (0.28 mmol, 1 eq.), 0.066 g of EDC (0.34 mmol, 1.2 eq.), 0.039 g of HOBt (0.28 mmol, 1 eq.) and 0.06 mL of diisopropylethylamine (0.37 mmol, 1.3 eq.) are successively placed in 1.5 mL of dioxane in a microwave tube. The medium is heated by microwave at 80°C for 45 minutes. The medium is evaporated. The residue is chromatographed on silica gel, eluting with a gradient of methanol in dichloromethane that varies from 1% to 3%. The fractions of interest are evaporated and the residue is washed with ethanol. 0.050 g of methyl *cis*-4-]4-[*cis*-4-(4-methoxycarbonylcyclohexyloxy)benzoyl(6-phenylaminopyridazin-3-yl)aminocarbonyl]phenoxy}cyclohexanecarboxylate is obtained.
M+H⁺ = 706.

### 21.2 Synthesis of methyl cis-4-{4-[6-(phenylamino)pyridazin-3-ylcarbamoyl]phenoxy}-cyclohexanecarboxylate

0.05 g of methyl *cis*-4-{4-[*cis*-4-(4-methoxycarbonylcyclohexyloxy)benzoyl(6-phenylaminopyridazin-3-yl)aminocarbonyl]phenoxy}cyclohexanecarboxylate (0.07 mmol, 1 eq.) and 0.02 mL of hydrazine hydrate (0.42 mmol, 6 eq.) are placed in 0.3 mL of pyridine. After stirring for 5 days, the medium is evaporated and diluted with dichloromethane. The organic phase is washed using a saturated solution of sodium hydrogencarbonate. The aqueous phase is extracted 3 times using dichloromethane. The organic phases are combined, dried over sodium sulfate, filtered and evaporated. The residue is chromatographed on silica gel, eluting with a gradient of methanol in dichloromethane that varies from 1% to 2%. 0.04 g of methyl *cis*-4-{4-[6-(phenylamino)-pyridazin-3-ylcarbamoyl]phenoxy}cyclohexanecarboxylate is obtained.
M+H⁺ = 447.

### 21.3 Synthesis of cis-4-[4-(6-phenylaminopyridazin-3-ylcarbamoyl)phenoxy]cyclo-hexanecarboxylic acid

0.04 g of methyl *cis*-4-{4-[6-(phenylamino)pyridazin-3-ylcarbamoyl]phenoxy}-cyclohexanecarboxylate (0.09 mmol, 1 eq.) is placed in 2 mL of tetrahydrofuran and 0.011 g of lithium hydroxide monohydrate (0.27 mmol, 3 eq.) dissolved in 1 mL of water is added. After stirring for 18 h, the medium is acidified using a 6% aqueous sulfurous acid solution, and filtered. The precipitate is triturated successively in water and ethanol to give 0.018 g of *cis*-4-[4-(6-phenylaminopyridazin-3-ylcarbamoyl)phenoxy]cyclohexanecarboxylic acid.
M+H⁺ = 433.
¹H NMR (400 MHz, DMSO-d₆) δ ppm 1.60 to 1.89 (m, 8 H); 2.39 (m, 1 H); 4.67 (m, 1 H); 6.94 (t, ***J=*7.8** Hz, 1 H); 7.05 (d, ***J=*9.0** Hz, 2 H); 7.23 (d, *J*=9.5 Hz, 1 H); 7.30 (t, *J*=7.8 Hz, 2 H); 7.72 (d, *J*=7.8 Hz, 2 H); 8.03 (d, *J*=9.0 Hz, 2 H); 8.09 (d, *J*=9.5 Hz, 1 H); 9.23 (s, 1 H); 10.86 (s, 1 H); 12.00 (very broad unresolved m, 1 H).

### Example 22: cis-4-{4-[6-(4-methoxyphenyl)pyridazin-3-ylcarbamoyl]phenoxy}-cyclohexanecarboxylic acid (compound No. 12)

### 22.1. Synthesis of methyl cis-4-{4-r6-(4-methoxyphenyl)pyridazin-3-ylcarbamoyl]-phenoxy}cyclohexanecarboxylate

0.506 g of 6-(4-methoxyphenyl)pyridazin-3-ylamine (2.52 mmol, 2 eq.), 0.350 g of *cis-*4-(4-methoxycarbonylcyclohexyloxy)benzoic acid (2.52 mmol, 2 eq.), 0.289 g of EDC (1.51 mmol, 1.2 eq.), 0.170 g of HOBt (1.26 mmol, 1 eq.) are successively placed in 6 mL of dioxane in a microwave tube. The medium is heated by microwave at 80°C for 65 minutes. The medium is evaporated. The residue is chromatographed on silica gel, eluting with a gradient of methanol in dichloromethane that varies from 0% to 5%. The fractions of interest are evaporated and 0.580 g of methyl *cis*-4-{4-[6-(4-methoxyphenyl)pyridazin-3-ylcarbamoyl]phenoxy}cyclohexanecarboxylate is obtained.
M+H⁺ = 462.

### 22.2. Synthesis of cis-4-{4-[6-(4-methoxyphenyl)pyridazin-3-ylcarbamoyl]phenoxy}-cyclohexanecarboxylic acid

0.250 g of methyl *cis*-4-{4-[6-(4-methoxyphenyl)pyridazin-3-ylcarbamoyl]phenoxy}cyclohexanecarboxylate (0.54 mmol, 1 eq.) is placed in 13.5 mL of a 2/1 mixture of tetrahydrofuran/water and 0.068 g of lithium hydroxide monohydrate (1.63 mmol, 3 eq.) is added. After stirring for 18 h, the medium is acidified using a 6% aqueous sulfurous acid solution, and filtered. The precipitate is triturated successively in water and ethanol. The residue is slurried in ethanol at high temperature and filtered to give 0.150 g of *cis*-4-{4-[6-(4-methoxyphenyl)pyridazin-3-ylcarbamoyl]phenoxy}cyclohexanecarboxylic acid.
M+H⁺ = 448.
¹H NMR (400 MHz, DMSO-d₆) δ ppm 1.59 to 1.91 (m, 8 H); 2.40 (m, 1 H); 3.84 (s, 3 H); 4.69 (m, 1 H); 7.04 to 7.15 (m, 4 H); 8.02 to 8.12 (m, 4 H); 8.20 (d, *J*=9.3 Hz, 1 H); 8.40 (d, *J*=9.3 Hz, 1 H); 11.25 (s, 1 H); 12.00 (very broad unresolved m, 1 H).

**Tables I** and **II** that follow illustrate the chemical structures and the physical properties of a few compounds according to the invention, corresponding to the formula (I).

**Table I** illustrates compounds of formula (I) according to the invention for which **D** is a bond; these compounds are referred to hereinbelow as compounds of formula (A).

**Table II** illustrates compounds of formula (I) according to the invention for which **D** is an oxygen atom, **Z2** and **Z3** are absent, these compounds being referred to hereinbelow as compounds of formula (B).

In these tables:
- the compounds of **table I** are mainly in trans form or exclusively in trans form;
- the compounds of **table II** are exclusively in cis form;
- "-" in the "Z1" column or the "Z2" column or the "R1" column or the "R2" column indicates that the corresponding group is absent;
- "*" indicates the bonding atom;
- "m.p." represents the melting point of the compound, expressed in degrees Celsius (°C). "then dec" means "then decomposition of the compound";
- "MH⁺" represents the mass M+H of the compound, obtained by LC-MS (abbreviation for liquid chromatography-mass spectroscopy);
- "-" in the "MH+" or "m.p." columns indicates that the measurement was not taken.

**TABLE I**

| | | | | | | | | | | | | | |
|---|---|---|---|---|---|---|---|---|---|---|---|---|---|
| 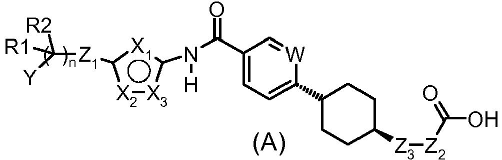 | | | | | | | | | | | | | |

| **No.** | **n** | **R1** | **R2** | **W** | **X1** | **X2** | **X3** | **Y** | **Z1** | **Z2** | **Z3** | **MH+** | **m**.**p**. |
|---|---|---|---|---|---|---|---|---|---|---|---|---|---|
| **1** | 1 | H | H | -CH- | N | O | N | 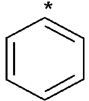 | - | - | -CH₂- | 420 | - |
| **2** | 1 | H | H | -CH- | N | N | O | 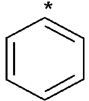 | - | - | -CH₂- | 420 | 234 |
| **3** | 1 | H | H | -CH- | -CH=CH- | N | N | 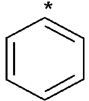 | - | - | -CH₂- | 430 | - |
| **3b** | 1 | H | H | -CH- | N | N | O | 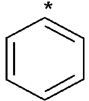 | - | 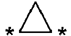 | | 446 | 187°C |
| **3c** | 1 | H | H | -CH- | N | N | O | 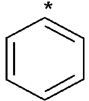 | - | -CH=CH- | | 432 | 210°C |
| **3d** | 1 | H | H | -CH- | N | N | O | 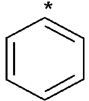 | - | -CH- | -CH- | 434 | 193°C |

**TABLE II**

| | | | | | | | | | | | |
|---|---|---|---|---|---|---|---|---|---|---|---|
| 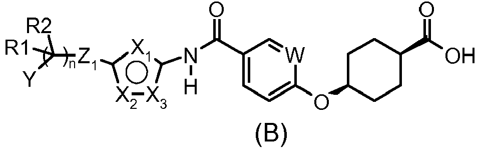 | | | | | | | | | | | |

| **No.** | **n** | **R1** | **R2** | **W** | **X1** | **X2** | **X3** | **Y** | **Z1** | **MH+** | **m**.**p**. |
|---|---|---|---|---|---|---|---|---|---|---|---|
| 4 | 1 | H | H | -CH- | N | N | O | 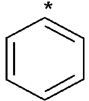 | - | 422 | 226 |
| **5** | 1 | H | H | -CH- | N | N | O | 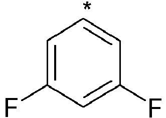 | - | 458 | 202 |
| **6** | 1 | 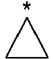 | | -CH- | N | N | O | 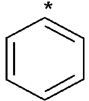 | - | 448 | 215-222 |
| **7** | 1 | -CH₃ | -CH₃ | -CH- | N | N | O | 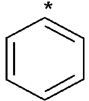 | - | 450 | 250-255 |
| 8 | 1 | H | H | -CH- | N | N | O | 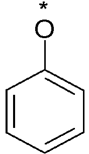 | - | 438 | 210-212 |
| **9** | 0 | - | - | -CH- | -CH=CH- | N | N | 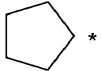 | -NH- | 425 | 215 then dec. |
| **10** | 1 | H | H | N | N | N | O | 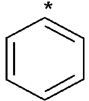 | - | 423 | 205-210°C |
| **11** | 0 | - | - | -CH- | -CH=CH- | N | N | 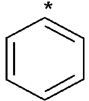 | -NH- | 433 | 259-261°C |
| **12** | 0 | - | - | -CH- | -CH=CH- | N | N | 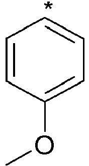 | - | 449 | 319-322°C |

The compounds according to the invention underwent pharmacological trials for determining their inhibitory effect on the DGAT-1 enzyme, which is involved in the metabolism of lipids.

These trials consisted in measuring the *in vitro* inhibitory activity of the compounds of the invention on the DGAT-1 enzyme by virtue of a phase partitioning assay (PPA) in 96-well format. The recombinant DGAT-1 protein was produced in Sf9 insect cells transfected with a baculovirus. The reaction was carried out by incubating the enzyme in the presence of 1,2-di(*cis*-9-octadecenoyl)-*sn*-glycerol and octanoylCoA. The amount of triacylglycerol formed during the reaction is measured. The inhibitory activity with respect to the DGAT-1 enzyme is given by the concentration that inhibits 50% of the activity of DGAT-1 (IC₅₀). The radioactivity is measured using a MicroBeta counter (Perkin Elmer).

The IC₅₀ values of the compounds according to the invention are less than 10 µM, more particularly between 100 nM and 1 µM, more particularly still less than 100 nM and more particularly between 50 and 100 nM. For example, the IC₅₀ values of compounds No. 1, 2, 3, 3b, 3c, 3d, 4, 5, 6, 7, 8, 9, 10, 11 and 12 are respectively 0.286, 0.253, 0.89, 0.742, 2.21, 0.927, 0.056, 0.115, 3.76, 5.7, 0.094, 0.051, 0.081, 5.91 and 7.18 µM.

It therefore appears that the compounds according to the invention have an inhibitory activity on the DGAT-1 enzyme. The compounds according to the invention may therefore be used for preparing medicaments, in particular medicaments for inhibiting the DGAT-1 enzyme.

The compounds according to the invention underwent pharmacological trials for determining their inhibitory effect on triglyceride biosynthesis.

These trials consisted in measuring the *in vitro* inhibitory activity of the compounds of the invention on a cell test.

Chang liver cells at 80% confluence are detached with trypsin-EDTA, 4 ml per 175 cm² flask. After centrifugation at 1300 × g for 5 minutes, the cell pellet is washed once with PBS and then resuspended in whole medium. The number of cells and their viability are determined on Mallassez cells via the exclusion method with trypan blue.

150 000 cells are inoculated per well into a 24-well plate for a minimum of 3 h in DMEM medium 4.5 g/l of glucose supplemented with 10% FCS and with antibiotics, and are maintained at 37°C in an incubator with CO₂ (5%).

After 3 h, the cells have adhered, the medium is removed and replaced overnight with DMEM medium 4.5 g/l of glucose with 2% of BSA/oleate.

After culturing for 18 h without serum, the test compounds are incubated for 30 min (1.3, 10, 30, 100, 300 and 1000 nM) with the cells, followed by addition of [¹⁴C] glycerol (0.4 µCi/ml/well) for an incorporation time of 6 h.

The supernatant is drawn off and the cells are recovered by treatment with trypsin-EDTA, 100 µl/well, for 5 minutes at 37°C. This cell suspension is then recovered in an Eppendorf tube and is washed with twice 500 µl of PBS. A centrifugation at 1300 × *g* for 5 minutes allows recovery of the cell pellet, which may be frozen at -20°C. In order to extract the lipids from the cell pellet, 400 µl of a methanol/dichloromethane/trifluoroacetic acid mixture (50/50/0.1%) is used to resuspend the cells. Next, the cell membranes are destroyed by sonication on a water bath, for 30 minutes. The samples are filtered through a 0.45 µm filter and then injected onto a C18 HPLC column of 4.6 × 75 mm, 3 µm with a mobile phase of 5% (H₂O + 0.1% TFA), 70% methanol, 25% dichloromethane, with a flow rate of 1.5 ml/minute. The radioactivity is measured using a Flo One C625TR machine (Perkin-Elmer).

The inhibitory activity on triglyceride biosynthesis is given by the concentration that inhibits 50% of the activity.

The activities of the compounds according to the invention are generally between 0.01 µM and 10 µM and more particularly between 0.01 and 1 µM.

For example, the activities of compounds Nos. 5 and 8 are, respectively, 0.310 and 0.319 µM.

It is thus seen that the compounds according to the invention have inhibitory activity on triglyceride biosynthesis.

The compounds according to the invention may thus be used for the preparation of medicaments, in particular medicaments for inhibiting triglyceride biosynthesis.

Thus, according to another of its aspects, a subject of the invention is medicaments comprising a compound of formula (I), or an addition salt thereof with a pharmaceutically acceptable acid or base of the compound of formula (I).

These medicaments find their use in therapeutics, especially in the treatment and/or prevention of obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome, coronary diseases, hypertension, skin diseases such as the skin diseases linked to the proliferation of the sebaceous glands such as acne, Alzheimer's disease, immunomodulatory diseases, HIV infection, irritable bowel syndrome and certain cancers, and advantageously for the preparation of a medicament for treating or preventing obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome and hepatitis C.

According to another of its aspects, the present invention relates to pharmaceutical compositions comprising, as active principle, a compound according to the invention. These pharmaceutical compositions comprise an effective dose of at least one compound according to the invention, or a pharmaceutically acceptable salt of said compound, and also at least one pharmaceutically acceptable excipient. Said excipients are chosen, according to the desired pharmaceutical form and mode of administration, from the usual excipients known to those skilled in the art.

In the pharmaceutical compositions of the present invention for oral, sublingual, subcutaneous, intramuscular, intravenous, topical, local, intratracheal, intranasal, transdermal or rectal administration, the active principle of formula (I) above, or the possible salt thereof, may be administered in unit administration form, as a mixture with standard pharmaceutical excipients, to animals and to man for the prophylaxis or treatment of the above disorders or diseases.

The appropriate unit administration forms include oral forms such as tablets, soft or hard gel capsules, powders, granules and oral solutions or suspensions, sublingual, buccal, intratracheal, intraocular, intranasal and inhalation administration forms, topical, transdermal, subcutaneous, intramuscular or intravenous administration forms, rectal administration forms and implants. For topical application, the compounds according to the invention may be used in creams, gels, ointments or lotions.

By way of example, a unit administration form of a compound according to the invention in tablet form may comprise the following components:

| | |
|---|---|
| Compound according to the invention | 50.0 mg |
| Mannitol | 223.75 mg |
| Croscaramellose sodium | 6.0 mg |
| Corn starch | 15.0 mg |
| Hydroxypropyl methyl cellulose | 2.25 mg |
| Magnesium stearate | 3.0 mg |

There may be particular cases in which higher or lower dosages are appropriate; such dosages are not outside the scope of the invention. According to the usual practice, the dosage that is appropriate for each patient is determined by the doctor according to the mode of administration and the weight and response of said patient.

## Claims

1. A compound corresponding to the formula (I) 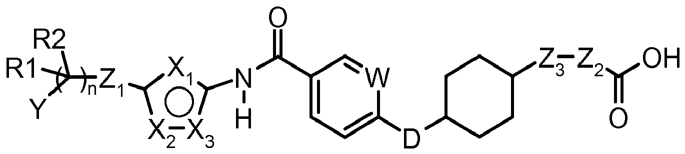 in which:
• **n** is equal to 0 or 1;
• **D** represents an oxygen atom or a bond;
• **W** represents a nitrogen atom or a -CH- group;
• **X1** represents a nitrogen atom or a -CH=CH- group;
• **X2** represents an oxygen atom or a nitrogen atom;
• **X3** represents an oxygen atom or a nitrogen atom; one of X1, X2, X3 being other than a nitrogen atom, X2 and X3 not being an oxygen atom at the same time;
• **R1, R2** are absent or represent,
o independently of one another, a hydrogen atom or a (C1-C4)alkyl group,
o R1 and R2 may form, with the carbon atom to which they are attached, a -(C3-C10)cycloalkyl- group;
• **Y** represents a -(C3-C10)cycloalkyl-, aryl or aryloxy group, said groups being optionally substituted with one or more substituents chosen from a halogen atom or a (C1-C6)alkoxy group;
• **Z1** is absent or represents an -NH- function;
• **Z2** is absent or represents a methylene group or a 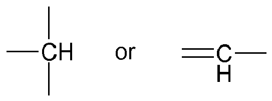 group;
**Z3** is absent or represents an oxygen atom or a methylene group or a 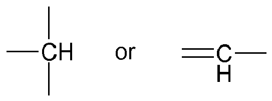 group;
given that **Z2** only represents a 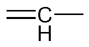 group when **Z3** is present and when it represents a 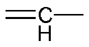 group, and vice versa, **Z2 and Z3** thus forming a double bond;
given that **Z2** and **Z3,** when they are present, may be included in a cycloalkyl group;
given that when **Z3** represents an oxygen atom, **Z2** represents a methylene group or a 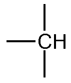 group;
in the form of an acid, a base or an addition salt with an acid or with a base.

2. The compound of formula (I) as claimed in claim 1, wherein:
• **n** is equal to 0 or 1;
• **D** represents an oxygen atom or a bond;
• **W** represents a -CH- group;
• **X1** represents a nitrogen atom or a -CH=CH- group;
• **X2** represents an oxygen atom or a nitrogen atom;
• **X3** represents an oxygen atom or a nitrogen atom; one of X1, X2, X3 being other than a nitrogen atom, X2 and X3 not being an oxygen atom at the same time;
• **R1, R2** are absent or represent,
o independently of one another, a hydrogen atom or a (C1-C4)alkyl group,
o R1 and R2 may form, with the carbon atom to which they are attached, a -(C3-C10)cycloalkyl- group;
• **Y** represents a -(C3-C10)cycloalkyl-, aryl or aryloxy group, said groups being optionally substituted with one or more substituents chosen from a halogen atom;
• **Z1** is absent or represents an -NH- function;
• **Z2** is absent;
• **Z3** is absent or represents a methylene group;
in the form of an acid, a base or an addition salt with an acid or with a base.

3. The compound of formula (I) as claimed in claim 1, wherein **X1** represents a nitrogen atom, **X2** and **X3** represent a nitrogen atom or an oxygen atom.

4. The compound of formula (I) as claimed in claim 1, wherein **X1** represents -CH=CH-, **X2** and **X3** represent a nitrogen atom.

5. The compound of formula (I) as claimed in one of the preceding claims, wherein it is chosen from the following compounds:
- trans{4-[4-(5-benzyl[1.2.4]oxadiazol-3-ylcarbamoyl)phenyl]cyclohexyl}acetic acid
- trans{4-[4-(3-benzyl[1.2.4]oxadiazol-5-ylcarbamoyl)phenyl]cyclohexyl}acetic acid
- cis-4-[4-(3-benzyl[1.2.4]oxadiazol-5-ylcarbamoyl)phenoxy]cyclohexanecarboxylic acid
- cis-4-{4-[3-(3,5-difluorobenzyl)[1.2.4]oxadiazol-5-ylcarbamoyl]phenoxy}cyclohexanecarboxylic acid
- cis-4-{4-[3-(1-phenylcyclopropyl)[1.2.4]oxadiazol-5-ylcarbamoyl]phenoxy}-cyclohexanecarboxylic acid
- cis-4-{4-[3-(1-methyl-1-phenylethyl)[1.2.4]oxadiazol-5-ylcarbamoyl]phenoxy}-cyclohexanecarboxylic acid
- cis-4-[4-(3-phenoxymethyl[1.2.4]oxadiazol-5-ylcarbamoyl)phenoxy]cyclohexanecarboxylic acid
- {4-[4-(6-benzylpyridazin-3-ylcarbamoyl)phenyl]cyclohexyl}acetic acid
- cis-4-[4-(6-cyclopentylaminopyridazin-3-ylcarbamoyl)phenoxy]cyclohexanecarboxylic acid
- cis-4-[5-(3-benzyl[1.2.4]oxadiazol-5-ylcarbamoyl)pyridin-2-yloxy]cyclohexane-carboxylic acid
- trans-2-{4-[4-(3-benzyl[1.2.4]oxadiazol-5-ylcarbamoyl)phenyl]cyclohexyl}-cyclopropanecarboxylic acid
- trans-(E)-3-{4-[4-(3-benzyl[1.2.4]oxadiazol-5-ylcarbamoyl)phenyl]cyclohexyl}-acrylic acid
- trans-3-{4-[4-(3-benzyl[1.2.4]oxadiazol-5-ylcarbamoyl)phenyl]cyclohexyl}-propionic acid
- cis-4-[4-(6-phenylaminopyridazin-3-ylcarbamoyl)phenoxy]cyclohexane-carboxylic acid
- cis-4-{4-[6-(4-methoxyphenyl)pyridazin-3-ylcarbamoyl]phenoxy}cyclohexanecarboxylic acid
in the form of an acid, a base or an addition salt with an acid or with a base.

6. A process for preparing a compound of formula (I) as claimed in any one of claims 1 to 5, wherein the ester function of a compound chosen from:
(i) a compound of formula (XXIX): 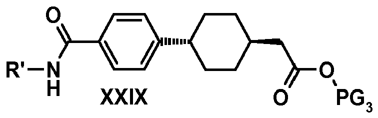 in which R' represents: 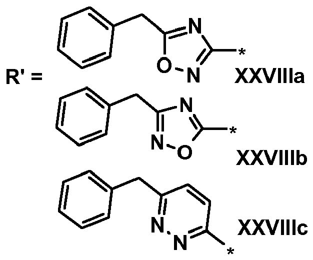 and PG3 represents a protecting group; and
(ii) a compound of formula (XXXI): 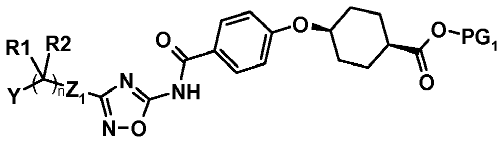 in which n, Y, Z1, R1, R2 and n are as defined in claim 1 and PG1 represents a protecting group; and
(iii) a compound of formula (XXXVI): 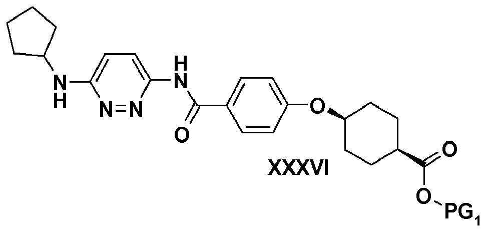 in which PG1 represents a protecting group, is deprotected.

7. The process for preparing a compound of formula (I) as claimed in claim 6, wherein the compound of general formula (XXXVI) is obtained by catalytic hydrogenation of a compound of formula (XXXV): 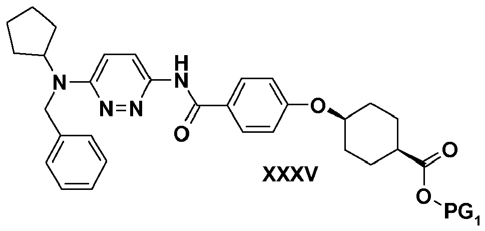 in which PG1 represents a protecting group.

8. The process for preparing a compound of formula (I) as claimed in claim 7, wherein the compound of general formula (XXXV) is obtained by reaction, with hydrazine NH₂-NH₂, of a compound of formula (XXXIV): 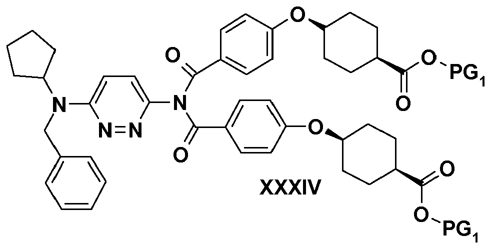 in which PG1 represents a protecting group.

9. Compounds of formula (XXIX), (XXXI), (XXXIV), (XXXV) and (XXXVI): 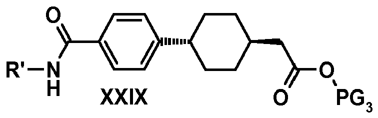 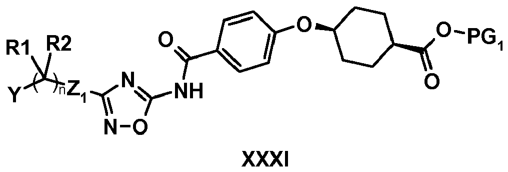 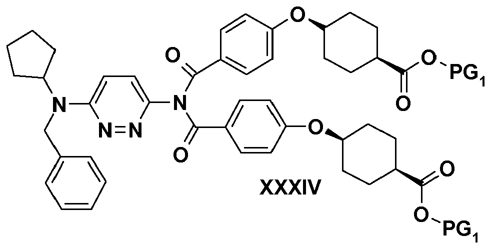 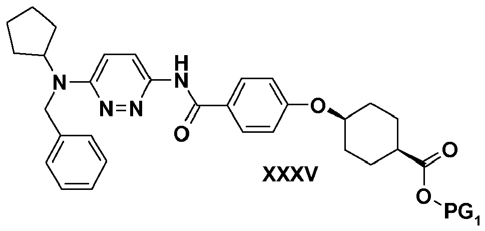 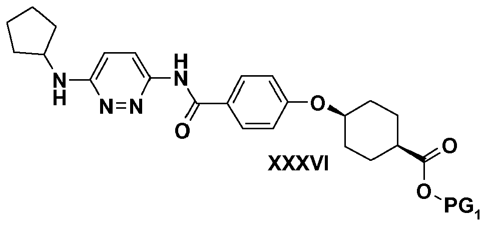 in which R' represents: 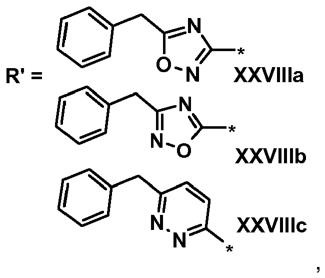 and n, Y, Z1, R1, R2 are as defined in claim 1 and PG1 and PG3 represent a protecting group.

10. A medicament, wherein it comprises a compound of formula (I) as claimed in any one of claims 1 to 5 or an addition salt of this compound with a pharmaceutically acceptable acid or base.

11. A pharmaceutical composition, wherein it comprises a compound of formula (I) as claimed in any one of claims 1 to 5 or a pharmaceutically acceptable salt of this compound, and also at least one pharmaceutically acceptable excipient.

12. The use of a compound of formula (I) as claimed in any one of claims 1 to 3, for the preparation of a medicament intended for treating and/or preventing any disease in which DGAT-1 is involved.

13. The use of a compound of formula (I) as claimed in claim 12, for the preparation of a medicament for treating and/or preventing obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome, coronary diseases, hypertension, skin diseases such as the skin diseases linked to the proliferation of the sebaceous glands such as acne, Alzheimer's disease, immunomodulatory diseases, HIV infection, irritable bowel syndrome and certain cancers, and advantageously for the preparation of a medicament intended for treating or preventing obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome and hepatitis C.

14. The compound of formula (I) as claimed in any one of claims 1 to 5, as a medicament.

15. The compound of formula (I) as claimed in any one of claims 1 to 5, for treating and/or preventing obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome, coronary diseases, hypertension, skin diseases such as the skin diseases linked to the proliferation of the sebaceous glands such as acne, Alzheimer's disease, immunomodulatory diseases, HIV infection, irritable bowel syndrome and certain cancers, and advantageously for treating or preventing obesity, dyslipidemia, impaired fasting glucose conditions, metabolic acidosis, ketosis, hepatic steatosis, insulin resistance, type 2 diabetes and complications arising from this pathology, lipotoxicity, the accumulation and an excess of triacylglycerides in adipose tissue (WAT), metabolic syndrome and hepatitis C.

## Patentansprüche

1. Verbindung der Formel (I) 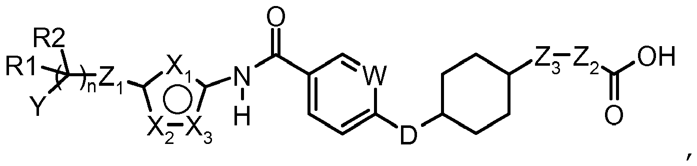 in welcher
• **n** gleich 0 oder 1 ist;
• **D** für ein Sauerstoffatom oder eine Bindung steht;
• **W** für ein Stickstoffatom oder eine -CH-Gruppe steht;
• **X1** für ein Stickstoffatom oder eine -CH=CH-Gruppe steht;
• **X2** für ein Sauerstoffatom oder ein Stickstoffatom steht;
• **X3** für ein Sauerstoffatom oder ein Stickstoffatom steht; wobei einer der Reste X1, X2, X3 nicht für ein Stickstoffatom steht und X2 und X3 nicht gleichzeitig für ein Sauerstoffatom stehen;
• **R1, R2** fehlen oder
o unabhängig voneinander für ein Wasserstoffatom oder eine (C1-C4)-Alkylgruppe stehen,
o R1 und R2 zusammen mit dem Kohlenstoffatom, an das sie gebunden sind, eine -(C3-C10)-Cycloalkylgruppe bilden können;
• **Y** für eine -(C3-C10)-Cycloalkyl-, Aryl- oder Aryloxygruppe steht, wobei diese Gruppen gegebenenfalls durch einen oder mehrere aus einem Halogenatom und einer (C1-C6)-Alkoxygruppe ausgewählte Substituenten substituiert sind;
• **Z1** fehlt oder für eine -NH-Funktion steht;
• **Z2** fehlt oder für eine Methylengruppe oder eine 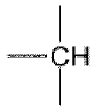 oder eine 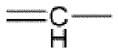 -Gruppe steht;
• **Z3** fehlt oder für ein Sauerstoffatom oder eine Methylengruppe oder eine 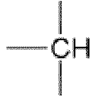 oder eine 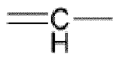 Gruppe steht;
mit der Maßgabe, dass **Z2** nur für eine 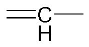 -Gruppe steht, wenn **Z3** vorhanden ist und wenn es für eine 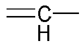 -Gruppe steht, und umgekehrt, so dass **Z2** und **Z3** eine Doppelbindung bilden;
mit der Maßgabe, dass **Z2** und **Z3,** wenn sie vorhanden sind, Teil einer Cycloalkylgruppe sein können;
mit der Maßgabe, dass, wenn **Z3** für ein Sauerstoffatom steht, **Z2** für eine Methylengruppe oder eine 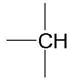 -Gruppe steht;
in Form einer Säure, einer Base oder eines Additionssalzes mit einer Säure oder mit einer Base.

2. Verbindung der Formel (I) nach Anspruch 1, wobei:
• **n** gleich 0 oder 1 ist;
• **D** für ein Sauerstoffatom oder eine Bindung steht;
• **W** für eine -CH-Gruppe steht;
• **X1** für ein Stickstoffatom oder eine -CH=CH-Gruppe steht;
• **X2** für ein Sauerstoffatom oder ein Stickstoffatom steht;
• **X3** für ein Sauerstoffatom oder ein Stickstoffatom steht; wobei einer der Reste X1, X2, X3 nicht für ein Stickstoffatom steht und X2 und X3 nicht gleichzeitig für ein Sauerstoffatom stehen;
• **R1, R2** fehlen oder
o unabhängig voneinander für ein Wasserstoffatom oder eine (C1-C4)-Alkylgruppe stehen,
o R1 und R2 zusammen mit dem Kohlenstoffatom, an das sie gebunden sind, eine -(C3-C10)-Cycloalkylgruppe bilden können;
• **Y** für eine -(C3-C10)-Cycloalkyl-, Aryl- oder Aryloxygruppe steht, wobei diese Gruppen gegebenenfalls durch einen oder mehrere aus einem Halogenatom ausgewählte Substituenten substituiert sind;
• **Z1** fehlt oder für eine -NH-Funktion steht;
• **Z2** fehlt;
• **Z3** fehlt oder für eine Methylengruppe steht;
in Form einer Säure, einer Base oder eines Additionssalzes mit einer Säure oder mit einer Base.

3. Verbindung der Formel (I) nach Anspruch 1, wobei **X1** für ein Stickstoffatom steht und **X2** und **X3** für ein Stickstoffatom oder ein Sauerstoffatom stehen.

4. Verbindung der Formel (I) nach Anspruch 1, wobei **X1** für -CH=CH- steht und **X2 und X3** für ein Stickstoffatom stehen.

5. Verbindung der Formel (I) nach einem der vorhergehenden Ansprüche, ausgewählt aus den folgenden Verbindungen:
- trans-{4-[4-(5-Benzyl-[1,2,4]oxadiazol-3-ylcarbamoyl)phenyl]cyclohexyl}essigsäure
- trans-{4-[4-(3-Benzyl-[1,2,4]oxadiazol-5-ylcarbamoyl)phenyl]cyclohexyl}essigsäure
- cis-4-[4-(3-Benzyl-[1,2,4]oxadiazol-5-ylcarbamoyl)phenoxy]cyclohexancarbonsäure
- cis-4-{4-[3-(3,5-Difluorbenzyl)-[1,2,4]oxadiazol-5-ylcarbamoyl]phenoxy}cyclohexancarbonsäure
- cis-4-{4-[3-(1-Phenylcyclopropyl)-[1,2,4]oxadiazol-5-ylcarbamoyl]phenoxy}cyclohexancarbonsäure
- cis-4-{4-[3-(1-Methyl-1-phenylethyl)-[1,2,4]oxadiazol-5-ylcarbamoyl]phenoxy}cyclohexancarbonsäure
- cis-4-[4-(3-Phenoxymethyl-[1,2,4]oxadiazol-5-ylcarbamoyl)phenoxy]cyclohexancarbonsäure
- {4-[4-(6-Benzylpyridazin-3-ylcarbamoyl)phenyl]cyclohexyl}essigsäure
- cis-4-[4-(6-Cyclopentylaminopyridazin-3-ylcarbamoyl)phenoxy]cyclohexancarbonsäure
- cis-4-[5-(3-Benzyl-[1,2,4]oxadiazol-5-ylcarbamoyl)pyridin-2-yloxy]cyclohexancarbonsäure
- trans-2-{4-[4-(3-Benzyl-[1,2,4]oxadiazol-5-ylcarbamoyl)phenyl]cyclohexyl}cyclopropancarbonsäure
- trans-(E)-3-{4-[4-(3-Benzyl-[1,2,4]oxadiazol-5-ylcarbamoyl)phenyl]cyclohexyl}acrylsäure
- trans-3-{4-[4-(3-Benzyl-[1,2,4]oxadiazol-5-ylcarbamoyl)phenyl]cyclohexyl}propionsäure
- cis-4-[4-(6-Phenylaminopyridazin-3-ylcarbamoyl)phenoxy]cyclohexancarbonsäure
- cis-4-{4-[6-(4-Methoxyphenyl)pyridazin-3-ylcarbamoyl]phenoxy}cyclohexancarbonsäure
in Form einer Säure, einer Base oder eines Additionssalzes mit einer Säure oder mit einer Base.

6. Verfahren zur Herstellung einer Verbindung der Formel (I) nach einem der Ansprüche 1 bis 5, bei dem man die Esterfunktion einer Verbindung ausgewählt aus:
(i) einer Verbindung der Formel (XXIX): 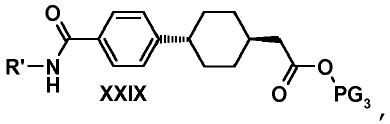 in welcher R` wie folgt definiert ist: 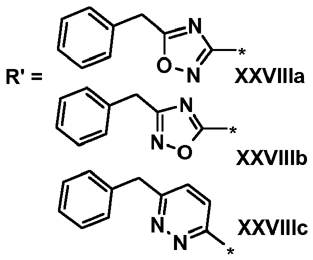 und PG3 für eine Schutzgruppe steht, und
(ii) einer Verbindung der Formel (XXXI): 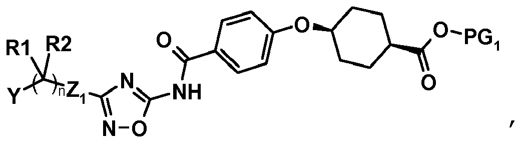 in welcher n, Y, Z1, R1, R2 und n wie in Anspruch 1 definiert sind und PG1 für eine Schutzgruppe steht, und
(iii) einer Verbindung der Formel (XXXVI): 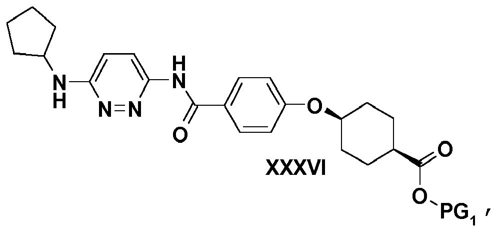 in welcher PG1 für eine Schutzgruppe steht, entschützt.

7. Verfahren zur Herstellung einer Verbindung der Formel (I) nach Anspruch 6, bei dem man die Verbindung der allgemeinen Formel (XXXVI) durch katalytische Hydrierung einer Verbindung der Formel (XXXV): 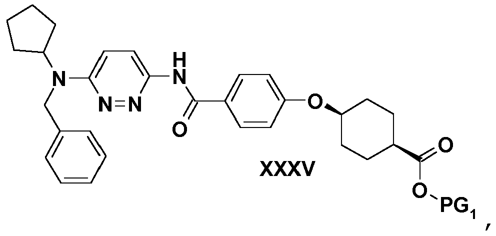 in welcher PG1 für eine Schutzgruppe steht, erhält.

8. Verfahren zur Herstellung einer Verbindung der Formel (I) nach Anspruch 7, bei dem man die Verbindung der allgemeinen Formel (XXXV) durch die Umsetzung einer Verbindung der Formel (XXXIV): 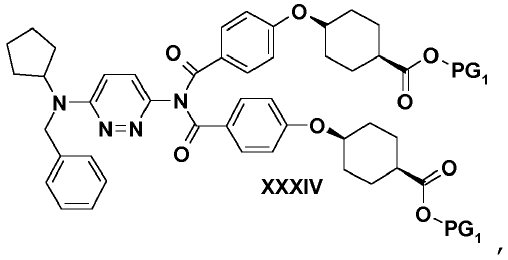 in welcher PG1 für eine Schutzgruppe steht, mit Hydrazin NH₂-NH₂ erhält.

9. Verbindungen der Formeln (XXIX), (XXXI), (XXXIV), (XXXV) und (XXXVI) : 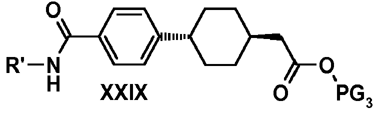 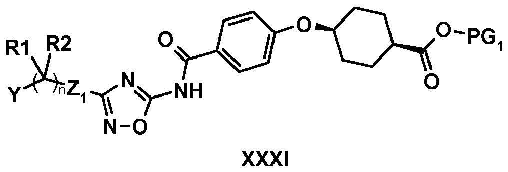 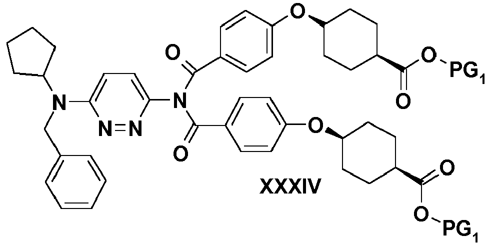 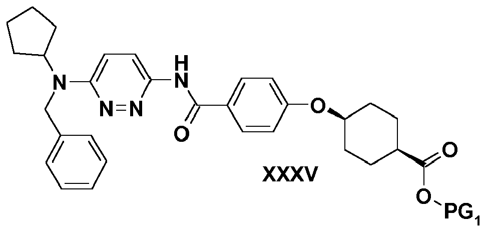 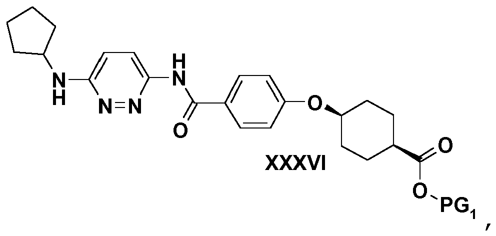 in welchen R` wie folgt definiert ist: 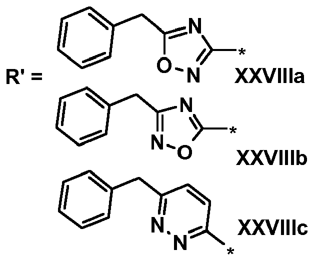 und n, Y, Z1, R1, R2 wie in Anspruch 1 definiert sind und PG1 und PG3 für eine Schutzgruppe stehen.

10. Medikament, welches eine Verbindung der Formel (I) nach einem der Ansprüche 1 bis 5 oder ein Additionssalz dieser Verbindung mit einer pharmazeutisch akzeptablen Säure oder Base umfasst.

11. Pharmazeutische Zusammensetzung, welche eine Verbindung der Formel (I) nach einem der Ansprüche 1 bis 5 oder ein pharmazeutisch akzeptables Salz dieser Verbindung und außerdem mindestens einen pharmazeutisch akzeptablen Exzipienten umfasst.

12. Verwendung einer Verbindung der Formel (I) nach einem der Ansprüche 1 bis 3 zur Herstellung eines für die Behandlung und/oder Prävention einer Krankheit, an der DGAT-1 involviert ist, vorgesehenen Medikaments.

13. Verwendung einer Verbindung der Formel (I) nach Anspruch 12 zur Herstellung eines Medikaments zur Behandlung und/oder Prävention von Obesitas, Dyslipidämie, gestörten Nüchternglucosebedingungen, metabolischer Azidose, Ketose, hepatischer Steatose, Insulinresistenz, Typ-II-Diabetes und von dieser Pathologie herrührenden Komplikationen, Lipotoxizität, der Anreicherung und einem Überschuss von Triacylglyceriden in Fettgewebe (WAT), metabolischem Syndrom, koronaren Herzkrankheiten, Hypertonie, Hautkrankheiten, wie den mit der Proliferation der Talgdrüsen verbundenen Hautkrankheiten wie Akne, Alzheimer-Krankheit, immunmodulatorischen Krankheiten, HIV-Infektion, Reizdarmsyndrom und bestimmten Krebsarten und vorteilhaft zur Herstellung eines für die Behandlung oder Prävention von Obesitas, Dyslipidämie, gestörten Nüchternglucosebedingungen, metabolischer Azidose, Ketose, hepatischer Steatose, Insulinresistenz, Typ-II-Diabetes und von dieser Pathologie herrührenden Komplikationen, Lipotoxizität, der Anreicherung und einem Überschuss von Triacylglyceriden in Fettgewebe (WAT), metabolischem Syndrom und Hepatitis C vorgesehenen Medikaments.

14. Verbindung der Formel (I) nach einem der Ansprüche 1 bis 5 als Medikament.

15. Verbindung der Formel (I) nach einem der Ansprüche 1 bis 5 zur Behandlung und/oder Prävention von Obesitas, Dyslipidämie, gestörten Nüchternglucosebedingungen, metabolischer Azidose, Ketose, hepatischer Steatose, Insulinresistenz, Typ-II- Diabetes und von dieser Pathologie herrührenden Komplikationen, Lipotoxizität, der Anreicherung und einem Überschuss von Triacylglyceriden in Fettgewebe (WAT), metabolischem Syndrom, koronaren Herzkrankheiten, Hypertonie, Hautkrankheiten, wie den mit der Proliferation der Talgdrüsen verbundenen Hautkrankheiten wie Akne, Alzheimer-Krankheit, immunmodulatorischen Krankheiten, HIV-Infektion, Reizdarmsyndrom und bestimmten Krebsarten und vorteilhaft zur Herstellung eines für die Behandlung oder Prävention von Obesitas, Dyslipidämie, gestörten Nüchternglucosebedingungen, metabolischer Azidose, Ketose, hepatischer Steatose, Insulinresistenz, Typ-II- Diabetes und von dieser Pathologie herrührenden Komplikationen, Lipotoxizität, der Anreicherung und einem Überschuss von Triacylglyceriden in Fettgewebe (WAT), metabolischem Syndrom und Hepatitis C vorgesehenen Medikaments.

## Revendications

1. Composé répondant à la formule (I) 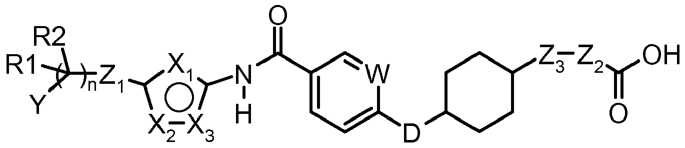 dans laquelle
• **n** est égal à 0 ou 1;
• **D** représente un atome d'oxygène ou une liaison ;
• **W** représente un atome d'azote ou un groupe -CH- ;
• **X1** représente un atome d'azote ou un groupe -CH=CH- ;
• **X2** représente un atome d'oxygène ou un atome d'azote ;
• **X3** représente un atome d'oxygène ou un atome d'azote ; l'un des X1, X2, X3 étant différent d'un atome d'azote, X2 et X3 n'étant pas simultanément un atome d'oxygène ;
• **R1, R2** sont absents ou représentent,
o indépendamment l'un de l'autre, un atome d'hydrogène ou un groupe (C1-C4)alkyle,
o R1 et R2 peuvent former avec l'atome de carbone auquel ils sont rattachés un groupe -(C3-C10)cycloalkyle- ;
• **Y** représente un groupe -(C3-C10)cycloalkyle-, aryle ou aryloxy, lesdits groupes étant éventuellement substitués par un ou plusieurs substituants choisis parmi un atome d'halogène ou un groupe (C1-C6)alkoxy ;
• **Z1** est absent ou représente une fonction-NH- ;
• **Z2** est absent ou représente un groupe méthylène ou un groupe 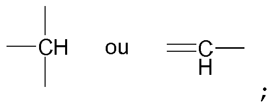
• **Z3** est absent ou représente un atome d'oxygène ou un groupe méthylène ou un groupe 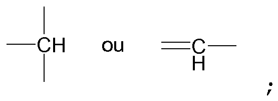 sachant que **Z2** ne représente un groupe 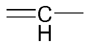 que lorsque **Z3** est présent et qu'il représente un groupe 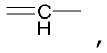 et réciproquement, **Z2 et Z3** formant ainsi une double liaison ;
sachant que **Z2** et **Z3,** lorsqu'ils sont présents, peuvent être compris dans un groupe cycloalkyle ;
sachant que lorsque **Z3** représente un atome d'oxygène, **Z2** représente un groupe méthylène ou un groupe 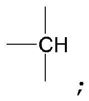 sous forme d'acide, de base ou de sel d'addition à un acide ou à une base.

2. Composé de formule (I) selon la revendication 1, **caractérisé en ce que**
• **n** est égal à 0 ou 1;
• **D** représente un atome d'oxygène ou une liaison ;
• **W** représente un groupe -CH- ;
• **X1** représente un atome d'azote ou un groupe -CH=CH- ;
• **X2** représente un atome d'oxygène ou un atome d'azote ;
• **X3** représente un atome d'oxygène ou un atome d'azote ; l'un des X1, X2, X3 étant différent d'un atome d'azote, X2 et X3 n'étant pas simultanément un atome d'oxygène ;
• **R1, R2** sont absents ou représentent :
o indépendamment l'un de l'autre, un atome d'hydrogène ou un groupe (C1-C4)alkyle,
∘ R1 et R2 peuvent former avec l'atome de carbone auquel ils sont rattachés un groupe -(C3-C10)cycloalkyle-;
• **Y** représente un groupe -(C3-C10)cycloalkyle-, aryle ou aryloxy, lesdits groupes étant éventuellement substitués par un ou plusieurs substituants choisis parmi un atome d'halogène ;
• **Z1** est absent ou représente une fonction -NH- ;
• **Z2** est absent ;
• **Z3** est absent ou représente un groupe méthylène ;
sous forme d'acide, de base ou de sel d'addition à un acide ou à une base.

3. Composé de formule (I) selon la revendication 1, **caractérisé en ce que X1** représente un atome d'azote, **X2** et **X3** représentent un atome d'azote ou un atome d'oxygène.

4. Composé de formule (I) selon la revendication 1, **caractérisé en ce que X1** représente -CH=CH-, **X2** et **X3** représentent un atome d'azote.

5. Composé de formule (I) selon l'une des revendications précédentes, **caractérisé en ce qu'**il est choisi parmi les composés suivants :
- acide trans-{4-[4-(5-benzyl-[1,2,4]oxadiazol-3-ylcarbamoyl)-phényl]-cyclohexyl}-acétique
- acide trans-{4-[4-(3-benzyl-[1,2,4]oxadiazol-5-ylcarbamoyl)-phényl]-cyclohexyl}-acétique
- acide cis-4-[4-(3-benzyl-[1,2,4]oxadiazol-5-ylcarbamoyl)-phénoxy]-cyclohexanecarboxylique
- acide cis-4-{4-[3-(3,5-difluoro-benzyl)-[1,2,4]oxadiazol-5-ylcarbamoyl]-phénoxy}-cyclohexanecarboxylique
- acide cis-4-{4-[3-(1-phényl-cyclopropyl)-[1,2,4]oxadiazol-5-ylcarbamoyl]-phénoxy}-cyclohexanecarboxylique
- acide cis-4-{4-[3-(1-méthyl-1-phényl-éthyl)-[1,2,4]oxadiazol-5-ylcarbamoyl]-phénoxy}-cyclohexanecarboxylique
- acide cis-4-[4-(3-phénoxyméthyl-[1,2,4]oxadiazol-5-ylcarbamoyl)-phénoxy]-cyclohexanecarboxylique
- acide {4-[4-(6-benzyl-pyridazin-3-ylcarbamoyl)-phényl]-cyclohexyl}-acétique
- acide cis-4-[4-(6-cyclopentylamino-pyridazin-3-ylcarbamoyl)-phénoxy]-cyclohexanecarboxylique
- acide cis-4-[5-(3-benzyl-[1,2,4]oxadiazol-5-ylcarbamoyl)-pyridin-2-yloxy]-cyclohexanecarboxylique
- acide trans-2-{4-[4-(3-benzyl-[1,2,4]oxadiazol-5-ylcarbamoyl)-phényl]-cyclohexyl}-cyclopropanecarboxylique
- acide trans-(E)-3-{4-[4-(3-benzyl-[1,2,4]oxadiazol-5-ylcarbamoyl)-phényl]-cyclohexyl}-acrylique
- acide trans-3-{4-[4-(3-benzyl-[1,2,4]oxadiazol-5-ylcarbamoyl)-phényl]-cyclohexyl}-propionique
- acide cis-4-[4-(6-phénylamino-pyridazin-3-ylcarbamoyl)-phénoxy]-cyclohexanecarboxylique
- acide cis-4-{4-[6-(4-méthoxy-phenyl)-pyridazin-3-ylcarbamoyl]-phénoxy}-cyclohexanecarboxylique
sous forme d'acide, de base ou de sel d'addition à un acide ou à une base.

6. Procédé de préparation d'un composé de formule (I) selon l'une quelconque des revendications 1 à 5, **caractérisé en ce que** l'on déprotège la fonction ester d'un composé choisi parmi
(i) un composé de formule (XXIX) 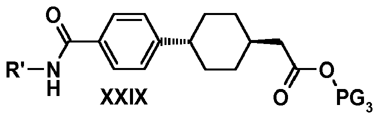 dans laquelle R' représente 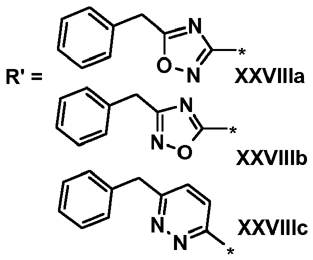 et PG3 représente un groupe protecteur ; et
(ii) un composé de formule (XXXI) : 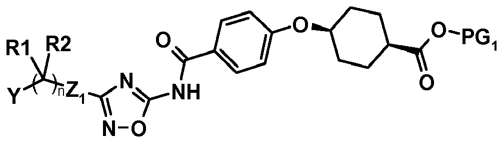 dans laquelle n, Y, Z1, R1, R2 et n sont tels que définis dans la revendication 1 et PG1 représente un groupe protecteur ; et
(iii)un composé de formule (XXXVI) 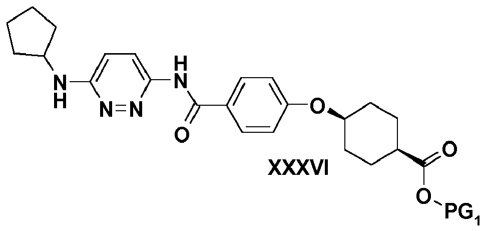 dans laquelle PG1 représente un groupe protecteur.

7. Procédé de préparation d'un composé de formule (I) selon la revendication 6, **caractérisé en ce que** le composé de formule générale (XXXVI) est obtenu par hydrogénation catalytique d'un composé de formule (XXXV) : 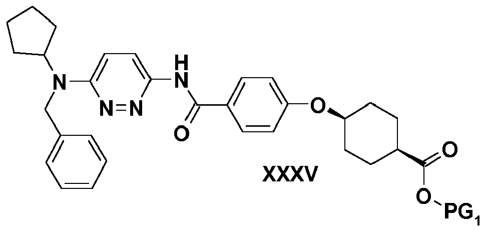 dans laquelle PG1 représente un groupe protecteur.

8. Procédé de préparation d'un composé de formule (I) selon la revendication 7, **caractérisé en ce que** le composé de formule générale (XXXV) est obtenu par réaction avec de l'hydrazine NH₂-NH₂ d'un composé de formule (XXXIV) : 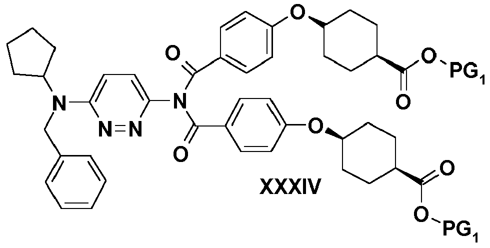 dans laquelle PG1 représente un groupe protecteur.

9. Composés de formule (XXIX), (XXXI), (XXXIV), (XXXV) et (XXXVI) : 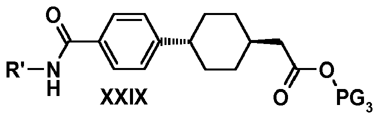 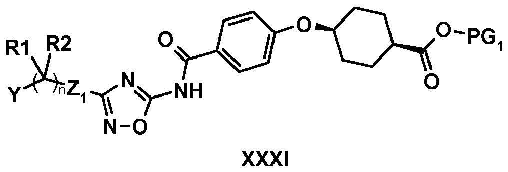 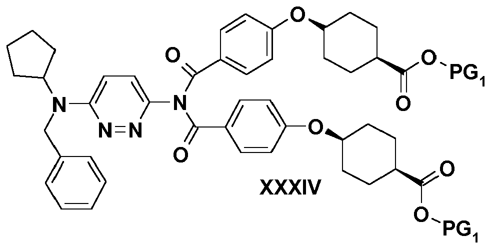 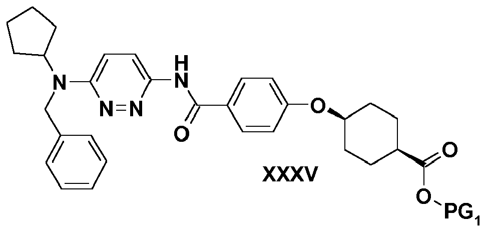 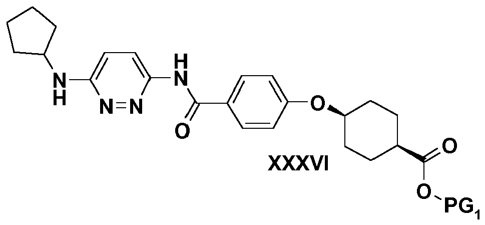 dans lesquelles R' représente 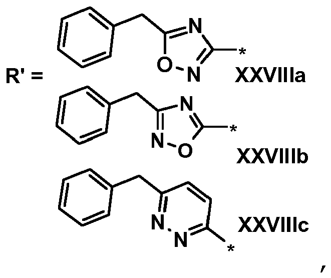 et n, Y, Z1, R1, R2 sont tels que définis dans la revendication 1 et PG1 et PG3 représentent un groupe protecteur.

10. Médicament, **caractérisé en ce qu'**il comprend un composé de formule (I) selon l'une quelconque des revendications 1 à 5, ou un sel d'addition de ce composé à un acide ou à une base pharmaceutiquement acceptable.

11. Composition pharmaceutique, **caractérisée en ce qu'**elle comprend un composé de formule (I) selon l'une quelconque des revendications 1 à 5, ou un sel pharmaceutiquement acceptable de ce composé, ainsi qu'au moins un excipient pharmaceutiquement acceptable.

12. Utilisation d'un composé de formule (I) selon l'une quelconque des revendications 1 à 3 pour la préparation d'un médicament destiné au traitement et/ou à la prévention de toute maladie dans laquelle DGAT-1 est impliqué.

13. Utilisation d'un composé de formule (I) selon la revendication 12 pour la préparation d'un médicament pour le traitement et/ou la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique, des maladies coronariennes, de l'hypertension, des maladies de peau telles que les maladies de peau liées à la prolifération des glandes sébacées telles que l'acné, de la maladie d'Alzheimer, des maladies immunomodulatrices, de l'infection à VIH, du syndrome inflammatoire de l'intestin et de certains cancers, et avantageusement pour la préparation d'un médicament destiné au traitement ou à la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique et de l'hépatite C.

14. Composé de formule (I) selon l'une quelconque des revendications 1 à 5 en tant que médicament.

15. Composé de formule (I) selon l'une quelconque des revendications 1 à 5 pour le traitement et/ou la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique, des maladies coronariennes, de l'hypertension, des maladies de peau telles que les maladies de peau liées à la prolifération des glandes sébacées telles que l'acné, de la maladie d'Alzheimer, des maladies immunomodulatrices, de l'infection à VIH, du syndrome inflammatoire de l'intestin et de certains cancers, et avantageusement pour le traitement ou la prévention de l'obésité, de la dyslipidémie, des conditions d'altération de glucose à jeun, de l'acidose métabolique, de la cétose, de la stéatose hépatique, de la résistance à l'insuline, du diabète de type 2 et des complications découlant de cette pathologie, de la lipotoxicité, de l'accumulation et de l'excès de triacylglycérides dans le tissu adipeux (WAT), du syndrome métabolique et de l'hépatite C.
